# EUROPEAN PATENT SPECIFICATION

(11) **EP 4 319 784 B1**
(45) Date of publication and mention of the grant of the patent: **24.07.2024**
(21) Application number: 22722163.7
(22) Date of filing: 08.04.2022
(51) Int. Cl.: A61K 36/48, A61K 31/00, A61K 31/14, A61P 11/00

(54) **PHARMACEUTICAL COMPOSITION FOR USE IN A TREATMENT OF VIRUS-INDUCED DISEASE**
PHARMAZEUTISCHE ZUSAMMENSETZUNG ZUR VERWENDUNG IN DER BEHANDLUNG VON VIRUSINDUZIERTEN ERKRANKUNGEN
COMPOSITION PHARMACEUTIQUE POUR L'UTILISATION DANS LE TRAITEMENT D'UNE MALADIE INDUITE PAR VIRUS

(30) Priority: 08.04.2021 DK PA202100349
(43) Date of publication of application: 14.02.2024
(73) Proprietor: QUR Medical ApS, 4622 Havdrup (DK)
(72) Inventor: OLSEN, Jens Steen, 4622 Havdrup (DK); TORNBY, Ditte Sandfeld, 5000 Odense C (DK); ANDERSEN, Thomas Emil, 5250 Odense SV (DK)
(74) Representative: Nordic Patent Service A/S
(86) International application number: PCT/EP2022/059468
(87) International publication number: WO 2022/214671

(56) References cited:
- BAHRAMSOLTANI ROODABEH ET AL: "An Evaluation of Traditional Persian Medicine for the Management of SARS-CoV-2", FRONTIERS IN PHARMACOLOGY, vol. 11, 25 November 2020 (2020-11-25), XP055948208, DOI: 10.3389/fphar.2020.571434
- SEN DEBANJAN ET AL: "Identification of potential inhibitors of SARS-CoV-2 main protease and spike receptor from 10 important spices through structure-based virtual screening and molecular dynamic study", JOURNAL OF BIOMOLECULAR STRUCTURE & DYNAMICS, vol. 40, no. 2, 18 September 2020 (2020-09-18), US, pages 941 - 962, XP055948075, ISSN: 0739-1102, Retrieved from the Internet <URL:https://www.tandfonline.com/doi/pdf/10.1080/07391102.2020.1819883> DOI: 10.1080/07391102.2020.1819883
- BAE MIN-JUNG ET AL: "Antiallergic effect of Trigonella foenum-graecum L. extracts on allergic skin inflammation induced by trimellitic anhydride in BALB/c mice", JOURNAL OF ETHNOPHARMACOLOGY, vol. 144, no. 3, 1 December 2012 (2012-12-01), IE, pages 514 - 522, XP055948177, ISSN: 0378-8741, DOI: 10.1016/j.jep.2012.09.030
- MUNIYAN ANBARASAN ET AL: "Characterization and in vitro antibacterial activity of saponin-conjugated silver nanoparticles against bacteria that cause burn wound infection", WORLD JOURNAL OF MICROBIOLOGY AND BIOTECHNOLOGY, SPRINGER NETHERLANDS, DORDRECHT, vol. 33, no. 7, 20 June 2017 (2017-06-20), pages 1 - 12, XP036273106, ISSN: 0959-3993, [retrieved on 20170620], DOI: 10.1007/S11274-017-2309-3

## Description

### TECHNICAL FIELD

The disclosure relates to a pharmaceutical composition suitable for treatment or prevention of a disease caused by a virus, in particular the SARS COVID-19 in any of its variants. The prevention of the disease includes administering the pharmaceutical composition to the patient before or after contact with an infected person.

### BACKGROUND

Most corona viruses cause common cold or mild airway infections. However, in 2003 a subgroup of corona virus emerged as the variant SARS-CoV, which resulted in the SARS epidemic in 2003. The epidemic entailed an outbreak totaling about 8000 infected individuals, including 800 dead in the period running for 2002 to 2003. Since 2004 there has been no reports of further incidences of the SARS-CoV infection.

In 2012 a new type of corona virus was detected and was named MERS-CoV. MERS-CoV belongs to the same subgroup of corona viruses as does SARS-CoV but is genetically distinguishable. Over 2,500 cases have been reported as of January 2021, including 45 cases in the year 2020. About 35% of those who are diagnosed with the disease die from it.

Coronavirus disease 2019 (COVID-19) was first identified in Wuhan, China, in December 2019. Severe acute respiratory syndrome coronavirus 2 (SARS-CoV-2) is a strain of coronavirus that causes COVID-19 (coronavirus disease 2019), the respiratory illness responsible for the ongoing COVID-19 pandemic. SARS-CoV-2 is a virus of the species severe acute respiratory syndrome-related coronavirus (SARSr-CoV), related to the SARS-CoV-1 virus that caused the 2002-2004 SARS outbreak. It is of zoonotic origins and has close genetic similarity to bat coronaviruses, suggesting it emerged from a bat-borne virus. SARS-CoV-2 belongs to the broad family of viruses known as coronaviruses. It is a positive-sense single-stranded RNA (+ssRNA) virus, with a single linear RNA segment and a lipid membrane envelope.

Coronaviruses infect humans, other mammals, including livestock and companion animals, and avian species.

Human coronaviruses are capable of causing illnesses ranging from the common cold to more severe diseases such as Middle East respiratory syndrome (MERS, fatality rate ~34%). SARS-CoV-2 is the seventh known coronavirus to infect people, after 229E, NL63, OC43, HKU1, MERS-CoV, and the original SARS-CoV.

A viral envelope is the outermost layer of many types of viruses. It protects the genetic material in their life cycle when traveling between host cells.

Viral envelopes are typically derived from portions of the host cell membranes (phospholipids and proteins) but include some viral glycoproteins. Envelopes may help viruses avoid the host immune system. Glycoproteins on the surface of the envelope serve to identify and bind to receptor sites on the host's membrane. The viral envelope then fuses with the host's membrane, allowing the capsid and viral genome to enter and infect the host. All enveloped viruses also have a capsid, another protein layer, between the envelope and the genome.

Enveloped viruses possess great adaptability and can change in a short time in order to evade the immune system. Enveloped viruses can cause persistent infections.

Many other enveloped viruses, such as HBV, HCV, HIV and influenza viruses, are pathogenic to humans and of clinical importance. Therefore, a need to develop suitable treatment methods against enveloped virus-induced diseases exists.

Influenza A and B viruses are members of the genus Influenzavirus classified within the family of enveloped viruses Orthomyxoviridae. Strains of Influenza A virus infect a wide variety of animal species including humans, horses, pigs, marine mammals and birds, whereas strains of Influenza B virus only appear to infect people.

In humans, Infections with Influenza A and B viruses can cause acute and sometimes severe respiratory disease in immunocompetent and immunocompromised persons. Viral infection with Influenza A and B occur in annual epidemics, often with rapid disease development and spread the infection. These epidemics can occur in small, localized outbreaks or as worldwide epidemics depending on the type of prevalent strain. Periodically, as a result of the continuous evolution of Influenza A viruses, disease epidemics occur which may have a significant impact on world health.

Transmission of influenza virus infection occurs by inhalation of virus-containing droplets from symptomatic or respiratory secretions of asymptomatic carriers. Conditions in the environment such as the crowd promotes infection transmission. Virus replication takes place in the cilium-bearing cylindrical epithelial cells in the upper and lower airways and results in necrosis and rejection of the cells.

The biggest virus release takes place from 1 day before to 3-4 days after disease onset.

During the course of an influenza epidemic, the prevalent virus strain may be present in 15-50% of the respiratory infections that occur in adults and children. The spectrum of disease can vary from mild upper respiratory tract disease to severe pneumonia. Acute pneumonia due to Influenza A or B viruses can be fatal, especially when they occur concomitantly with secondary microbial infections in
elderly or immunocompromised patients.

Influenza viruses have been associated with nosocomial outbreaks of respiratory infections in pediatric (pediatric) and geriatric ("elderly") wards and led to prolonged hospitalization and increased morbidity and mortality.

Symptoms of COVID-19 are varied, but often include fever, cough, fatigue, breathing difficulties, and loss of smell and taste. Symptoms begin within one to fourteen days after exposure to the virus. Of those people who develop noticeable symptoms, most (81%) develop mild to moderate symptoms (up to mild pneumonia), while 14% develop severe symptoms (dyspnea, hypoxia, or more than 50% lung involvement on imaging), and 5% suffer critical symptoms (respiratory failure, shock, or multiorgan dysfunction). At least a third of the people who are infected with the virus are asymptomatic and do not develop noticeable symptoms at any point in time, but they still can spread the disease.

The virus that causes COVID-19 spreads mainly when an infected person is in close contact with another person. Small droplets and aerosols containing the virus can spread from an infected person's nose and mouth as they breathe, cough, sneeze, sing, or speak. Other people are infected if the virus gets into their mouth, nose or eyes. The virus may also spread via contaminated surfaces, although this is not thought to be the main route of transmission. The exact route of transmission is rarely proven conclusively, but infection mainly happens when people are near each other for long enough. People who are infected can transmit the virus to another person up to two days before they themselves show symptoms, as can people who do not experience symptoms. People remain infectious for up to ten days after the onset of symptoms in moderate cases and up to 20 days in severe cases.

The COVID-19 leaves individuals in uncertainty as to whether they are infected because the latency period may be as high as 14 days. Furthermore, many infected persons are asymptomatic. Thus, the individual may be spreading the virus to other persons without awareness. Also, some people are not willing to get vaccinated or to have their children vaccinated. Thus, health authorities are in need of an alternative to vaccination, which is commonly used to control the pandemic.

Herpes simplex virus 1 and 2 (HSV-1 and HSV-2), also known by their taxonomical names Human alphaherpesvirus 1 and Human alphaherpesvirus 2, are two members of the human Herpesviridae family, a set of new enveloped viruses that produce viral infections in the majority of humans. Both HSV-1 (which produces most cold sores) and HSV-2 (which produces most genital herpes) are common and contagious. They can be spread when an infected person begins shedding the virus.

About 67% of the world population under the age of 50 has HSV-1. About 47.8% and 11.9% are believed to have HSV-1 and HSV-2, respectively. Because it can be transmitted through any intimate contact, it is one of the most common sexually transmitted infections.

The human immunodeficiency viruses (HIV) are two enveloped species of Lentivirus (a subgroup of retrovirus) that infect humans. Over time, they cause acquired immunodeficiency syndrome (AIDS), a condition in which progressive failure of the immune system allows life-threatening opportunistic infections and cancers to thrive. Without treatment, average survival time after infection with HIV is estimated to be 9 to 11 years, depending on the HIV subtype.

Further, vaccine approaches against HIV and HSV have failed so far and high costs of antiviral treatment exclude a large part of the world population from treatment. We need ways to restrict the virus spread, including ways for women to protect themselves from HIV-1 and HSV. HSV-2 infection is co-factor for HIV-1 shedding and acquisition and control of HSV-2 is suggested as a mean of reducing HIV-1 transmission.

A very early stage of viral infection is viral entry when the virus attaches to and enters the host cell. Several "entry-inhibiting" or "entry-blocking" drugs are being developed to fight HIV. HIV most heavily targets the immune system's white blood cells known as "helper T cells" and identifies these target cells through T-cell surface receptors designated "CD4" and "CCR5". Attempts to interfere with the binding of HIV with the CD4 receptor have failed to stop HIV from infecting helper T cells, but research continues on trying to interfere with the binding of HIV to the CCR5 receptor in hopes that it will be more effective.

Cytomegalovirus (CMV) is a genus of enveloped viruses in the order Herpesvirales, in the family Herpesviridae, in the subfamily Betaherpesvirinae. Humans and monkeys serve as natural hosts. The 11 species in this genus include human betaherpesvirus 5 (HCMV, human cytomegalovirus, HHV-5), which is the species that infects humans. Diseases associated with HHV-5 include mononucleosis and pneumonia. In the medical literature, most mentions of CMV without further specification refer implicitly to human CMV.

Roodabeth B. et al. "An evaluation of Traditional Persian Medicine" published 25 Nov. 2020, provides a review of a medical effect of traditional Persian plants, among them fenugreek (i.e. Trigonella foenum-graecum) and its use for treating a virus-induced disease. Sen Debanjan et al.: "Identification of potential inhibitors of SARS-CoV-2 main protease and spike receptor from 10 important spices through structure-based virtual screening and molecular dynamic study", JOURNAL OF BIOMOLECULAR STRUCTURE & DYNAMICS, vol. 40, no. 2, 18 September 2020 (2020-09-18), discloses compounds found in fenugreek have been demonstrated to possess an inhibitory effect on SARS-CoV-2 main protease and spike receptor in in-vitro assays.

### SUMMARY

The present invention is defined by the claims.

It is an object to provide a pharmaceutical composition for use in a method of the treatment of a virus-induced disease, wherein the composition comprises an extract of Trigonella foenum-graecum and optionally pharmaceutically acceptable additives, wherein the method comprises administration of between 20mg and 250mg Trigonella foenum-graecum extract at least once daily.

Trigonella foenum-graecum (also termed Fenugreek or TFG herein) is an annual herb belonging to the legume family. The TFG seed is a major constituent of curry and a part of traditional Indian and Asian cooking. TFG is considered safe as a human food component, taste enhancer, and coloring agent. The TFG seed is rich in phytochemicals, including proteins, steroidal saponin, flavanoids, tannic acids, stearic acid, vegetal oils, alkaloide trigonelline and 4-hydroxyisoleucine.

The extract of TFG used in the pharmaceutical composition of the present invention is likewise considered safe and can be used as a human food component. Therefore, the pharmaceutical composition of the present invention can be administrated freely the patient without risk concerns. As with all chemical compounds, a toxic level exists, however, it has not been detected yet.

The present invention advantageously offers people that have not yet been vaccinated or are reluctant to being vaccinated against diseases for which vaccines currently exist an alternative to traditional vaccines. The prevention or treatment of,e.g.,COVID-19 allows asymptomatic persons to advantageously safely engage in social and job-related activities in groups comprising individuals vulnerable to the disease without fearing of spreading the virus.

The foregoing and other objects are achieved by the features of the independent claims. Further implementation forms are apparent from the dependent claims and the description.

According to a first aspect, there is provided a pharmaceutical composition for use in a method for the treatment of virus-induced disease, comprising an extract of Trigonella foenum-graecum and optionally pharmaceutically acceptable additives.

In a possible implementation form of the first aspect the virus is an enveloped virus, Coronavirus, a Simplexvirus, a Cytomegalovirus, an Orthomixovirus, and/or a Retrovirus. These genera are enveloped viruses, i.e., viruses that have a lipid membrane.

In a possible implementation form of the first aspect the virus is a Severe Acute Respiratory Syndrome-related Coronavirus (SARSr-CoV), such as SARS-CoV-1 or SARS-CoV-2, a Herpes Simplex Virus 1 or 2, a Human Betaherpesvirus 5 (HCMV, human cytomegalovirus, HHV-5), an Influenza A, B, C or D virus and/or a Human Immunodeficiency Virus 1 or 2 (HIV-1, HIV-2).

In a possible implementation form of the first aspect said SARSr-CoV is a genetic lineage variant of concern (VOC), a variant under monitoring, or variant of interest (VOI) as defined by the World Health Organisation. A VOC is known as a SARS-CoV-2 variant that meets the definition of a VOI (see below) and, through a comparative assessment, has been demonstrated to be associated with one or more of the following changes at a degree of global public health significance:
- Increase in transmissibility or detrimental change in COVID-19 epidemiology; OR
- Increase in virulence or change in clinical disease presentation; OR
- Decrease in effectiveness of public health and social measures or available diagnostics, vaccines, therapeutics.

A VOI is known as a SARS-CoV-2 variant :
- with genetic changes that are predicted or known to affect virus characteristics such as transmissibility, disease severity, immune escape, diagnostic or therapeutic escape; and
- Identified to cause significant community transmission or multiple COVID-19 clusters, in multiple countries with increasing relative prevalence alongside increasing number of cases over time, or other apparent epidemiological impacts to suggest an emerging risk to global public health.

A VUM is known as a SARS-CoV-2 variant with genetic changes that are suspected to affect virus characteristics with some indication that it may pose a future risk, but evidence of phenotypic or epidemiological impact is currently unclear, requiring enhanced monitoring and repeat assessment pending new evidence.

It is expected that understanding of the impacts of variants may fast evolve, and designated Variants under Monitoring may be readily added/removed. See https://www.who.int/en/activities/tracking-SARS-CoV-2-variants/?msclkid=6740ed46b67611ec92afe7354be42af5 for more details.

In a possible implementation form of the first aspect the SARSr-CoV virus is a SARS-CoV-2 wildtype or an Alpha (B.1.1.7), Beta (B.1.351), Gamma (P.1), Delta (B.1.617.2), Mink (Cluster N439K/Y453F variant), and/or Omicron (B.1.1.529), Epsilon (B.1.427/B.1.429), Zeta (P.2), Eta (B.1.525), Theta (P.3), Iota (B.1.526), Kappa (B.1.617.1), Lambda (C.37), or Mu (B.1.621) genetic lineage variant.

In a possible implementation form of the first aspect the disease is a Severe Acute Respiratory Syndrome caused by SARS-CoV-1 or SARS-CoV-2, such as COVID-19, cold sores, caused by Herpes Simplex Virus 1, genital herpes, caused by Herpes Simplex Virus 2, mononucleosis, pneumonia, both caused by Human Betaherpesvirus 5, influenza (flu), caused by Influenza A, B, C and/or D viruses, and/or acquired immunodeficiency syndrome (AIDS), caused by Human Immunodeficiency Virus 1 or 2.

In a possible implementation form of the first aspect the pharmaceutical composition further comprises bentonite. In some formulations of the pharmaceutical composition the mixture of the extract of Trigonella foenum-graecum and bentonite behaves synergistically, i.e. the effect of the extract is potentiated by the presence of bentonite.

In a possible implementation form of the first aspect the pharmaceutical composition the bentonite comprises 50% by weight or more of smectite.

In a possible implementation form of the first aspect the the pharmaceutical composition comprises 1:10 to 10:1 by weight dry matter of Trigonella foenum-graecum extract to bentonite.

In an embodiment of the invention the weight of Trigonella foenum-graecum extract in the pharmaceutical composition is at least 0.01% by weight of the pharmaceutical composition.

In a possible implementation form of the first aspect the weight of Trigonella foenum-graecum extract is at most approximately 30% by weight of the pharmaceutical composition, such as at most approximately 25% by weight of the pharmaceutical composition, such as at most approximately 15% by weight of the pharmaceutical composition, such as at most approximately 5% by weight of the pharmaceutical composition, such as at most approximately 1% by weight of the pharmaceutical composition, such as at most approximately 0.5% by weight of the pharmaceutical composition, such as at most approximately 0.25% by weight of the pharmaceutical composition, such as at most approximately 0.15% by weight of the pharmaceutical composition.

The pharmaceutical composition of the invention may formulated in any way suitable for the path of administration. In a certain implementation of the pharmaceutical composition, it is formulated as a solution for spraying, a topical medication, such as a cream or a lotion, a throat lozenge, or a tablet. When the pharmaceutical composition is administrated to a mucous membrane it is generally in the form of a spray or at throat lozenge. A spray is usually preferred for administration to a mucous membrane of the lung and the nasal regions, whereas a throat lozenge or an orodispersible tablet are a suitable administration forms for infections present in the throat. Generally, the solution for spraying is for nasal or pulmonal administration.

According to an implementation of the pharmaceutical composition the throat lozenge or tablet is an orodispersible tablet.

In a certain implementation form of the first aspect, the orodispersible tablet, throat lozenge or tablet is administered sublingually or buccally. Suitably, any of these sublingually administered forms are dissolved during a time period of 1 to 20 minutes.

In a possible implementation form of the first aspect the pharmaceutical composition comprises at least 20mg Trigonella foenum-graecum extract, such as at least 30mg Trigonella foenum-graecum extract, such as at least 40mg Trigonella foenum-graecum extract, such as at least 50mg Trigonella foenum-graecum extract, such as at least 60mg Trigonella foenum-graecum extract, such as at least 70mg Trigonella foenum-graecum extract, such as at least 80mg Trigonella foenum-graecum extract, such as at least 900mg Trigonella foenum-graecum extract, such as at least 100mg Trigonella foenum-graecum extract, such as at least 110mg Trigonella foenum-graecum extract, such as at least 120mg Trigonella foenum-graecum extract, such as at least 130mg Trigonella foenum-graecum extract, such as at least 140mg Trigonella foenum-graecum extract, such as at least 150mg Trigonella foenum-graecum extract, such as at least 160mg Trigonella foenum-graecum extract, such as at least 170mg Trigonella foenum-graecum extract, such as at least 180mg Trigonella foenum-graecum extract, such as at least 190mg Trigonella foenum-graecum extract, such as at least 200mg Trigonella foenum-graecum extract, such as at least 210mg Trigonella foenum-graecum extract, such as at least 220mg Trigonella foenum-graecum extract, such as at least 230mg Trigonella foenum-graecum extract, such as at least 240mg Trigonella foenum-graecum extract, such as 250mg Trigonella foenum-graecum extract.

In a possible implementation form of the first aspect the pharmaceutical composition is administered at least once daily, such as two times daily, such as three times daily, such as four times daily, such as five times daily.

In a possible implementation form of the first aspect the pharmaceutical composition advantageously diminishes the ability of the virus to replicate. Such a mechanism might include one or more of diminishing the ability of the virus to attach to a host cell, penetrate the host cell, inhibition of nucleic acid transcription in the host cell, and/or virus release and, thus, cell to cell spreading.

In a possible implementation form of the first aspect the pharmaceutical composition advantageously diminishes the ability of the virus to attach to a host cell. Such a strategy is one of anti-viral strategies relying on interference with the ability of a virus to infiltrate a target cell. The virus must go through a sequence of steps to penetrate a host cell, beginning with binding or attaching to a specific "receptor" molecule on the surface of the host cell and ending with the virus "uncoating" inside the cell and releasing its contents. Viruses that have a lipid envelope must also fuse their envelope with the target cell, or with a vesicle that transports them into the cell before they can uncoat.

The attaching stage of viral replication can be inhibited in two ways:
1. Using agents which mimic the virus-associated protein (VAP) and bind to a cellular receptor. This may include Trigonella foenum-graecum extract components according to the invention, which may advantageously act as natural ligands of the receptor.
2. Using agents which mimic the cellular receptor and bind to the VAP. This may include Trigonella foenum-graecum extract components according to the invention, which may advantageously act as extraneous receptor mimics.

In a possible implementation form of the first aspect the pharmaceutical composition advantageously diminishes the ability of the virus to penetrate a host cell. A very early stage of viral infection is viral entry, when the virus attaches to and enters the host cell. HIV, for example, infects a cell through fusion with the cell membrane, which requires two different cellular molecular participants, CD4 and a chemokine receptor (differing depending on the cell type). Potentially, one of the benefits from the use of an effective entry-blocking or entry-inhibiting agent is that it potentially may not only prevent the spread of the virus within an infected individual but also the spread from an infected to an uninfected individual. Another possible advantage of the therapeutic approach of blocking viral entry (as opposed to the currently dominant approach of viral enzyme inhibition) is that it may prove more difficult for the virus to develop resistance to this therapy than for the virus to mutate or evolve its enzymatic protocols.

Anti-penetration agents act by blocking a pocket on the surface of the virus that controls the uncoating/penetration process. This pocket is similar in most strains of influenza, for example.

In a possible implementation form of the first aspect the pharmaceutical composition advantageously diminishes the ability of the lipid membrane to remain intact. In such an event, decreased integrity of the viral envelope may lead to the appearance of holes and/or reduced ability of fusing with the cellular membrane of a host cell, which may advantageously diminish the ability to attach and penetrate a host cell. Further, envelopes may help viruses avoid the host immune system, thus, and without the wish to be bound by any particular hypothesis, decreased integrity of the viral envelope may lead to decreased ability of the virus to evade the immune system, thereby leading to remission of the disease.

In a possible implementation form of the first aspect the pharmaceutical composition advantageously diminishes the ability of the virus to replicate and/or transcribe its genetic material within the host cell. Trigonella foenum-graecum extract components according to the invention may act as nucleotide or nucleoside analogues, which may advantageously deactivate the enzymes that synthesize the RNA or DNA once the analogue is incorporated, and/or inhibit integrase enzymes that integrate the synthesized DNA into the host cell genome.

In a possible implementation form of the first aspect the pharmaceutical composition advantageously diminishes the ability of the virus to translate and correctly process nascent peptides within the host cell.

In a possible implementation form of the first aspect the pharmaceutical composition advantageously targets long dsRNA helixes.

In a possible implementation form of the first aspect the pharmaceutical composition advantageously diminishes the ability of the virus to release completed viruses from the host cell. Suitably, blocking a molecule named neuraminidase that is found on the surface of flu viruses, and also seems to be constant across a wide range of flu strains, may advantageously prevent the release of viral particles.

In a possible implementation form of the first aspect the pharmaceutical composition advantageously effects a change on a cellular membrane of a host cell. Such a change may comprise a conformational change of a proteic membrane component, a change in the membrane potential of said membrane or an increase in permeability of said membrane to ions and/or molecules. These changes may lead to a decrease in the attachment, penetration and/or release of vira.

The trigonella foenum-graecum extract may be obtained by any method known by the person skilled in the art for extracting components from plant materials. In a second aspect of the invention the extract is obtainable by the steps of:
a) contacting Trigonella foenum-graecum seeds and a solvent in a mixture,
b) incubating said mixture for at least 2 hours, such as for at least 3 hours, until the embryo is released from the seeds
c) optionally heating said mixture, and
d) recovering a liquid extract from said mixture by separating remaining plant material from the mixture.

Suitably, the incubation in step b) may be continued until the sprouting is visible.

The method for preparing the extract may further comprise the step of removing the solvent by spray drying. Thus, the Trigonella foenum-graecum extract in the pharmaceutical composition may be spray dried particles.

In a possible implementation form of the second aspect, the method further comprises
f) freezing prior to or after the heating in c) for at least 3 hours, preferably more than 6 hours, such as 12 hours, and
g) optionally, heating the blend before recovery of the extract.

### DETAILED DESCRIPTION

### Extract

A method for preparation of an extract from Trigonella foenum-graecum may be found in WO 08/125120. According to the disclosure of the prior art publication, seeds of trigonella foenum-graecum are submerged in water to initiate sprouting before the extraction. WO 17/207010 discloses a composition comprising a mixture of trigonella foenum-graecum extract and bentonite. WO 08/125120 and WO 17/207010 are incorporated herein by reference.

In one aspect of the invention the extract of trigonella foenum-graecum is derived from plant material, such as leaves, branches, flowers, seeds etc. In a suitable embodiment said plant material is seeds obtained from trigonella foenum-graecum.

The method of preparing said extract may comprise the following steps:
a) contacting Trigonella foenum-graecum plant material and a solvent in a mixture,
b) incubating said mixture for at least 2 hours, such as for at least 3 hours, until the embryo is released from the seeds
c) optionally heating said mixture, and
d) recovering a liquid extract from said mixture by separating remaining plant material from the mixture.

The plant material may be fresh, frozen, dried, or combinations thereof. In the preferred embodiment the plant material is seeds of Trigonella foenum-graecum, most preferably dried seeds of said plant.

In order to facilitate the extraction of the active ingredients of the plant material, said plant material is soaked in a solvent, preferably water. The blend of liquid and plant material is incubated for at least 2 hours, more preferably at least 3 hours, preferably at least 6 hours, even more preferably at least 12 hours, such as at least 24 hours. The incubation is usually performed at temperatures between 0 and 45°C, suitably at temperatures between 10 and 40°C. The incubation should preferably continue at least until the sprouting is visible.

Subsequently, the blend comprising the plant material soaked in a liquid is heated, preferably to a temperature above the coagulation of proteins. In a certain aspect the blend is boiled.

The blend comprises plant material and a fluid solvent. The ratio by weight of said plant material and said liquid in said blend is suitably 1 to 1, or preferably less plant material by weight such as 1 to 2, or less plant material by weight such as 1 to 3, or less plant material by weight such as 1 to 4, or less plant material by weight such as 1 to 5, or less plant material by weight such as 1 to 6, or less plant material by weight such as 1 to 7, or less plant material by weight such as 1 to 8, or less plant material by weight such as 1 to 9, or less plant material by weight such as 1 to 10. In a preferred embodiment the ratio by weight of said plant material and said liquid is 1 to 6.

During the heating of the blend additional fluid may be added at least once in order to compensate for evaporated liquid and liquid taken up by the plant material. The liquid is preferably heated. The heating may be terminated when the embryo is released from the seeds, which is associated with increased viscosity of the blend. Suitably, the heating is not continued more than 10 minutes after the embryo has been released.

Optionally, the blend is frozen (preferably at -18°C) prior to or after the heating step for at least 3 hours, preferably more than 6 hours, such as 12 hours, or more than 12 hours. Subsequently, the blend may be subjected to a second round of heating before recovery of the extract, e.g. by removing the remaining plant material. The freezing step is expected to further enhance the release of the active ingredients from the plant material.

The volume of a final concentrated extract originating from ½ kg of plant material such as seeds is approximately 2 liters.

For conservation, the extract may be refrigerated. Depending on the application the extract may be diluted in water or used as is. The extract may be further concentrated by removal of solvent. The solvent may be removed or reduced by any appropriate means, such as membrane filtration, evaporation, precipitation, extraction, azeotrope distillation, lyophilisation, spray drying and combinations thereof. The spray dried particles may subsequently be post-dried in a fluid bed apparatus.

The aqueous extract of trigonella foenum-graecum has a tendency to smell of sotolon. The smell may be considered unpleasant by some users and may therefore restrict the application of the extract. The present inventors have surprisingly found that the amount of sotolon may be reduced drastically by spray drying the extract. Therefore, the aqueous extract is preferably dried by spray drying to form a fenugreek powder.

The extract used in the invention may be purified to isolate the active ingredient(s) by any appropriate method. Thus, the extract may be fractioned using gel filtration, HPCL, extraction, precipitation, etc. In a presently useful method, the extract is fractioned using HPLC. In a specific method the active ingredient(s) is included in an extract fraction obtainable by performing reverse phase chromatography on a size B Lichroprep RP-18 (40-63um) (Merck) of the basis extract using the following gradient: 0-1 min H₂O/AcN 98:2, then using a steady gradient from 1-40 min going to 100% and collecting the fraction at the time interval between 5 and 10 min. The solvent of the purified extract may be removed or reduced by any of the methods disclosed above. In an aspect of the invention, the purified extract is spray dried.

In another specific purification method, the aqueous extract may initially be treated with ethanol to precipitate the majority of the plant residues and polysaccharides from the extract. The precipitate may be removed by sedimentation or centrifugation. For easier storage, the solvent may be evaporated or otherwise removed so as to produce a powder. Alternatively, the ethanol treated extract may be used directly in the subsequent process. The powder may subsequently be suspended in water and acidified to pH 1-4, preferably pH 2, with a strong acid such as hydrochloric acid. The acidified extract is extracted with an organic water immiscible solvent like heptane. After agitation the organic and the aqueous layer are separated and the aqueous layer is treated with an alkaline agent to obtain a pH above pH 9, preferably around pH 10. The alkaline aqueous phase is again extracted with an organic water immiscible solvent and agitated. A solid powder is obtained from the recovered organic phase by removing the solvent by evaporation, such as by evaporation under reduced pressure or spray drying.

### Bentonite

Bentonite is an absorbent aluminum phyllosilicate clay. Bentonite is usually formed from weathering or diagenesis of volcanic ash. The different types of bentonite are generally named after the dominant ion, i.e. sodium bentonite, potassium bentonite, aluminum bentonite, iron bentonite, and calcium bentonite. Sodium and calcium bentonite are generally preferred.

Sodium bentonite is characterized by a high swelling power, often absorbing as much as several times its dry mass in water. Calcium bentonite is an adsorbent of ions and other components and is characterized by a somewhat lower swelling power. Calcium bentonite may have the calcium ion ex-changed with the sodium ion to convert it to sodium bentonite (termed sodium beneficiation or sodium activation) to exhibit many of sodium bentonite's properties by an ion exchange process. This transformation can be accomplished by adding a soluble sodium compound to the Ca-bentonite.

As bentonite is a naturally occurring clay, it may contain a complex blend of components. In general, the bentonite of the present invention comprises smectite clay. The smectite may e.g. be selected among montmorillonite, beidellite, sauconite, stevensite hectorite, saponite, nontronite, vermiculite, and mixtures thereof. Also present in the bentonite clay may be kaolin, illite, and/or chlorite. The amount of smectite in the bentonite is generally above 50% by weight, such as above 70 % by weight, and suitably above 80 % by weight.

Smectite is defined in clay mineralogy as a 2:1 clay - consisting of an octahedral sheet sandwiched between two tetrahedral sheets. Smectites are comprised of layers of negatively charged aluminosilicate sheets held together by charge - balancing counter-ions such as Na⁺ and Ca²⁺. In the presence of water these cations tend to hydrate, thereby forcing the clay layers apart in a series of discrete steps. This causes the smectite to swell.

It will be understood that the bentonite used in the composition of the present invention may be naturally occurring and unmodified bentonite, or any fraction thereof enriched in a certain component and optionally chemically modified, especially by exchange of ions. Montmorillonite is hydrated sodium calcium aluminum magnesium silicate hydroxide having the formula (Na,Ca)0.33(Al,Mg)2(Si4O1O)(OH)2·nH20. In one embodiment the bentonite clay is montmorillonite.

In aqueous environments, each smectite particle is composed of a multitude of submicroscopic platelets stacked in sandwich fashion with a layer of water between each. A single platelet is about one nanometer thick and up to several hundred nanometers across. Once the clay is hydrated, the weakly positive platelet edges are attached to the negatively charged platelet faces. A three-dimensional colloidal structure forms, which accounts for the characteristic rheology imparted by these clays, i.e., an increase in viscosity.

The amount of smectite in the composition is generally not above 50% by weight. For most practical purposes, an amount of approximately 30% by weight or less of bentonite clay is used to avoid a viscosity, which is not desirable from an end-user point of view. Suitably, the amount of smectite is 10% by weight or less, such as 5% by weight or less. To obtain a microorganism reducing effect even a small amount of bentonite is suitable. In general, the amount of smectite is 0.0001 % by weight or above, such as 0.001 % by weight, 0.01 % by weight, 0.1% or above and preferably 0.3% by weight and above.

Bentonite clays are commercially available under various trade names including Van Gel B, Veegum, Veegum F, Veegum HV, VeegumK, Veegum HS, Veegum Ultra, Veegum D, Veegum Pure, Veegum Ultra, Veegum PRO, Veegum plus, Veegum T, Van Gel B, Van Gel C, Van Gel ES, Van Gel O, all trademarks of R.T. Vandebilt Company. Bentonite clays are also available from Amcol International.

Smectite clay may also be provided synthetically e.g., following the method of Nakazawa, H., Yamada, H., and Fujita, T. (1992): Crystal synthesis of smectite applying very high pressure and temperature, Applied Clay Science, 6, 395-401.

Bentonite occurs in many geological areas of the world. According to British Geological Survey bentonite is produced in at least 44 countries. Thus, sodium bentonite is i.a. produced in USA, in South Dakota and in Wyoming. Sodium bentonite is also produced in Turkey in the Tokat Resadiye region. Mixed sodium/calcium bentonite is mined in Greece, Australia, India, Russia and Ukraine. Calcium bentonite is mined in Mississipi and Alabama, Germany, Greece, Turkey, India, and China. In a certain aspect of the invention a bentonite produced in Denmark near Rødby is preferred.

The Trigonella foenum-graecum extract and bentonite may be mixed in any proportion that provide the intended effect. In a certain aspect of the invention the Trigonella foenum-graecum extract to bentonite is mixed in a weight ratio between 1:10 to 10:1. Suitably, the weight ratio between Trigonella foenum-graecum extract to bentonite is at least 2:10, such as 3:10. Similarly, the weight ratio of bentonite to Trigonella foenum-graecum extract is preferably at least 2:10, such as 3:10. In a preferred aspect the weight ratio between Trigonella foenum-graecum extract to bentonite is between 4:10 and 10:4.

### Formulation

The composition may comprise a variety of further components in addition to the trigonella foenum-graecum extract. According to a certain aspect of the invention, when the pharmaceutical composition comprises bentonite, the weight of the mixture of trigonella foenum-graecum extract and bentonite is at least 0.01% by weight of the final formulation, such as at least 0.05% by weight and suitably at least 0.1% by weight.

The pharmaceutical composition according to the invention may be formulated in a number of different manners, depending on the purpose of the particular medicament and the type of administration. It is well within the scope of a person skilled in the arts to formulate compositions that are in accordance with the preferred type of administration.

The composition comprising the extract and optionally bentonite according to the invention may be prepared by any conventional technique, e.g., as described in Remington: The Science and Practice of Pharmacy 1995, edited by E. W. Martin, Mack Publishing Company, 19th edition, Easton, Pa.

The composition may comprise pharmaceutical acceptable additives such as any conventionally used pharmaceutical acceptable additive, which should be selected according to the specific formulation, intended administration route etc. For example, the pharmaceutically acceptable additives may be any of the additives mentioned in Nema et al, 1997. Furthermore, the pharmaceutical acceptable additive may be any accepted additive from FDA's "inactive ingredients list", which can be downloaded from https://www.fda.gov/drugs/drug-approvals-and-databases/inactive-ingredients-database-download.

One preferred embodiment of the present invention is to provide a pharmaceutical composition formulated for application on a mucosal area, such as the mucosa present in the nasal cavity, mouth cavity, larynx, trachea, and thoracic cavity.

Compositions for topical administration may include pharmaceutically acceptable components such as carriers, surfactants, preservatives, stabilizing agents, buffers, excipients and emulsifiers suited for this type of administration. Suitable components for topical delivery systems are preferably chosen from components that do not cause excessive or unavoidable irritation or pain to the recipient. Carriers include diluents and provide the medium in which the pharmaceutical constituents are dissolved, dispersed or distributed.

The composition according to the invention may comprise, but are not restricted to, a carrier such as an aqueous liquid base, emulsion or suspension of solid particles in a liquid.

The composition of the invention may be applied to a mucosal area and the active component(s) may exert their action on the mucosa or after penetration of the mucosa. The topical availability of active compounds depends on various factors including their ability to dissolve in the carrier (gel, cream - hydrophilic), and their ability to permeate the mucosa, thus requiring a certain hydrophobic-hydrophilic balance. Formulations may require addition of excipients, such as permeation enhancers and solubilizers to facilitate either or both of the transport processes (dissolution into vehicle and diffusion across mucosa). Additives, such as alcohols, fatty alcohols, fatty acids, mono- di- or tri-glycerides, glycerol monoethers, cyclodextrin and derivatives, polymers, bioadhesives, terpenes, chelating agents and surfactants have been disclosed to increase transmucosal delivery of drugs. Alcohols include, but are not limited to, ethanol, 2-propanol and polyols such as polyethylene glycol (PEG), propylene glycol, glycerol, propanediol. It is within the present invention to make use of such excipients.

Any method, not limited to the above-mentioned, for increasing transmucosal delivery is within the scope of the present invention. The medicament according to the present invention may therefore comprise surfactants such as ionic and/or non-ionic surfactants. Suitable non-ionic surfactants include for example: fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (Pluronic); carboxyvinyl polymer (Polygel^{®} HP), fatty acid alkylolamides (fatty acid amide polyethylene glycols); alkyl polyglycosides, N-alkyl-, N-alkoxypolyhydroxy fatty acid amide, in particular N-methyl-fatty acid glucamide, sucrose esters; sorbitol esters, esters of sorbitol polyglycol ethers and lecithin. Ionic surfactants include for example sodium lauryl sulfate, sodium laurate, polyoxyethylene-20-cetylether, Laureth-9, sodium dodecylsulfate (SDS) and dioctyl sodium sulfosuccinate.

Methods for enhancing drug delivery through mucosal administration may be applied with the present invention, and include any means of increasing absorption, minimizing metabolism, and/or prolonging the half-life of the active ingredient of the medicament such as the extract of trigonella foenum-graecum. Such means may include the use of transporters of the type liposomes, ISCOMs, nano-particles, microspheres, hydrogels, organogels, polymers or other micro-encapsulation techniques.

### Orodispersible tablets:

Orodispersible tablets have improved patient compliance and have some additional advantages compared to other oral formulation. They are also solid unit dosage forms, which disintegrate in the mouth within a minute in the presence of saliva due to super disintegrants in the formulation. Thus, this type of drug delivery helps a proper peroral administration in pediatric and geriatric population where swallowing is a matter of trouble. Various scientists have prepared orodispersible tablets by following various methods.

However, the most common method of preparation is the compression method. Other special methods are molding, melt granulation, phase-transition process, sublimation, freeze-drying, spray-drying, and effervescent method. Since these tablets dissolve directly in the mouth, their taste is also an important factor. Various approaches have been taken in order to mask the bitter taste of a drug. A number of scientists have explored several drugs in this field. Like all other solid dosage forms, they are also evaluated in the field of hardness, friability, wetting time, moisture uptake, disintegration test, and dissolution test.

Methods for preparation of orodispersible tablets include: Molding methods: Tablets formed by molding process are highly porous in structure, resulting in high rate of disintegration and dissolution. This process includes moistening, dissolving, or dispersing the drugs with a solvent then molding the moist mixture into tablets by applying lower pressure in compression molding, but always lower than the conventional tablet compression. The powder mixture may be sieved prior to the preparation in order to increase the dissolution. Molded tablets have low mechanical strength, which results in erosion and breakage during handling.

Compaction methods: Conventional methods for the preparation of tablets such as dry granulation, wet granulation, and direct compression are also exist for the preparation of orodispersible tablets. Some important super disintegrants, which are used during preparation of orodispersible tablets, are crosspovidone, crosscarmellose sodium, sodium alginate, acrylic acid derivatives. Sodium starch glycolate may be used as super disintegrants as well as 2%-10% microcrystalline cellulose and crosspovidone. It has been found that preparation by compression method along with addition of super disintegrants in correct concentration obey all the properties of orodispersible tablets.

Spray-drying method: Orodispersible tablets are made up of hydrolyzed or unhydrolyzed gelatin as supporting agent for matrix, mannitol as bulk agent, and sodium starch glycolate or croscarmellose sodium as disintegrating agent. In order to improve the disintegration and dissolution, citric acid and sodium bicarbonate are used. Finally, the formulation is spray-dried in a spray drier. Orodispersible tablets prepared through this method are disintegrated in less than 20s.

Freeze-drying method: This is a very popular process for the preparation of orodispersible tablets. Tablets prepared by this process have low mechanical strength, poor stability at higher temperature and humidity, but glossy amorphous structure resulting in highly porous, lightweight product. There are various patents on this particular technology.

Insofar the pharmaceutical composition is formulated as a solution for spraying or a lozenge, one or more of the following additives may be used in the formulation: ethanol, allantoin, ammonia solution, anhydrous citric acid, ascorbic acid, benzalkonium chloride, benzoic acid, benzyl alcohol, betadex, boric acid, butylated hydroxyanisole, C13-14 isoparaffin/laureth-7/polyacrylamide, arbomer copolymer type b (allyl pentaerythritol crosslinked), carbomer homopolymer, carbomer homopolymer type a (allyl pentaerythritol crosslinked), carbomer homopolymer type b (allyl pentaerythritol crosslinked), carbomer homopolymer type b (allyl sucrose crosslinked), carbomer homopolymer type c (allyl pentaerythritol crosslinked), carboxymethylcellulose sodium, castor oil, citric acid monohydrate, cocoyl caprylocaprate, collagen, cyclomethicone/dimethicone copolyol, d&c yellow no. 10, denatonium benzoate, diethylene glycol monoethyl ether, diisopropanolamine, diisopropyl adipate, dimethicone 100, disodium laureth sulfosuccinate, disodium lauryl sulfosuccinate, docusate sodium, edetate disodium, edetic acid, fd&c green no. 3, fd&c yellow no. 6, glycerin, glyceryl oleate, hexylene glycol, high density polyethylene, hyaluronate sodium, hydrochloric acid, hydroxyethyl cellulose, hydroxypropyl cellulose, hypromellose, isopropyl alcohol, isopropyl myristate, isopropyl palmitate, isostearic acid, lactic acid, laureth-4, lavender oil, lecithin, lemon oil, light mineral oil, limonene, mannitol, medium-chain triglycerides, menthol, methyl laurate, methyl salicylate, methylparaben, microcrystalline wax, mineral oil, niacinamide, octoxynol-9, octyldodecanol, oleyl alcohol, parfum creme 45/3, peg/ppg-18/18 dimethicone, peg-60 hydrogenated castor oil, peg-7 methyl ether, pentadecalactone, phenonip, phenoxyethanol, phosphoric acid, poloxamer, polycarbophil, polyethylene glycol, polyoxyl 20 cetostearyl ether, polypropylene glycol, polysorbate, potassium hydroxide, ppg-15 stearyl ether, ppg-20 methyl glucose ether distearate, propyl gallate, propylene glycol, propylene glycol monolaurate, propylparaben, ricinoleic acid, sodium saccharin, sd alcohol 40-2, sepineo p 600, silicon dioxide, sodium benzoate, sodium chloride, sodium hydroxide, sodium lactate, sodium lauryl sulfate, sodium phosphate, sorbic acid, sorbitan monolaurate, succinic acid, tert-butyl alcohol, titanium dioxide, trisodium citrate dihydrate, trolamine, tromethamine, tyloxapol, xanthan gum, ethanol, butylated hydroxytoluene, carbomer copolymer type c (allyl pentaerythritol crosslinked), carbomer homopolymer type c (allyl pentaerythritol crosslinked), isopropyl myristate, oleic acid, propylene glycol, and sodium hydroxide.

Throat lozenges are pharmaceutical compositions designed to dissolve in the mouth cavity, typically by sublingual or buccal administration. Preferably, the extract of fenugreek is administered together with bentonite to potentiate the pharmaceutical effect.

The pharmaceutical acceptable additives for the throat lozenge may include one or more of the following substances: acacia, acesulfame potassium, citric acid, aspartame, calcium polycarbofil, citric acid monohydrate, dextrates, dextrose, cinnamon flavor, peppermint falvor, hydroxypropyl cellulose, magnesium stearate, maltodextrin, mannitol, methyl salicylate, peppermint oil, potassium bicarbonate, povidone K30, silicon dioxide, sodium alginate, sodium bicarbonate, sodium carbonate, sodium phosphate, sodium stearyl fumarate, pregelatinized starch, sorbitol, sucralose, sucrose, talc, xanthan gum.

Preferably, when bentonite is present in the composition, the mixture of the extract of trigonella foenum-graecum and the bentonite is present in the same composition. Alternatively, they may be supplied in a kit of parts, i.e. one part comprises the extract of trigonella foenum-graecum and the other part comprises the bentonite.

The solution for spraying is administrated as an aerosol (or mist) to the relevant area of the body. The spraying may be performed by a so-called nebulizer commonly used in the treatment of respiratory diseases or disorders. Nebulizers use oxygen, compressed air or ultrasonic power to break up solutions and suspensions into small aerosol droplets that are inhaled from the mouthpiece of the device. Example of a useful nebulizer is Philips InnoSpire Essence Nebulizer and Beurer Inhalator.

The spraying may also be performed by an inhaler, such as a metered-dose inhaler (MDI), a dry powder inhaler, or a soft mist inhaler. Inhalers are designed to deliver medication directly to the lungs through a person's own breathing. This may benefit a patient by providing the extract directly to areas of disease, allowing medication to take a greater effect on its intended target, and limit side effects of medications due to localized treatment. The aerosols are typically below a size of 6 micrometer to allow them to be delivered to the alveoli.

Nasal sprays include a liquid pharmaceutical composition, which is insufflated through the nose. Suitable devices are mechanical liquid spray pumps and pressurized metered-dose inhalers (pMDIs) for nasal use. It may be desired to limit the fraction of respirable particles below 9 µm in order to avoid lung inhalation of pharmaceutical composition.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality.

### Examples

### Example 1

Preparation of an aerosol for oral administration An aqueous solution of the fenugreek powder is prepared by providing 1962g water measured in a beaker. During mild agitation 20,0g Versatil PC obtained from Evonik Dr. Straetmans GmbH was added together with 5,00g extract of fenugreek is prepared in example 3.

The aqueous solution was administrated to Covid-19 patients by a Philips InnoSpire Essence Nebulizer.

### Example 2

### Preparation of throat lozenges

The following components were added to a container during agitation to obtain a powder mixture:

| | |
|---|---|
| Fenugreek powder | 12.0 kg |
| Bentonite | 6.0 kg |
| Menthol | 1.33kg |
| Micronized silica gel*⁾ | 4.00 kg |
| Pepermint | 9.50 kg |
| Magnesium stearate | 11.00 kg |
| Sorbitol (granular) | 1,000 kg |

### ^{*)} Syloid AL-1

The power mixture was conveyed to a tablet press and tablets having an average weight of 1044 mg was obtained.

### Example 3

### Preparation of fenugreek powder

Preparation of a powder extract from Trigonella foenum-graecum (fenugreek) seeds was performed as follows: 500g seeds of Trigonella foenum-graecum were soaked in 2.5 l water for approximately 24 hours. Following the pre-soaking the seeds were cooked for 20 minutes and remains of the seeds were removed from the mixture.

The aqueous extract was spray dried for obtaining a powder in accordance with ISO9001:2008. The aqueous extract had a dry matter content of 1.2-2.0 % by weight and a temperature of 5°C. In a concentrate heater the extract was heated to a temperature of 62°C prior to spraying by a centrifugal atomizer (GEA Niro) running at 12.500 rpm. The dryer inlet temperature was 170 °C and the dryer outlet temperature was 87 °C. The spray dried powder was post-dried in a fluid bed at a temperature of 24 °C. The powder moisture content of the dried product was 3.58 % by weight and the size of the particles was mainly in the range of 5-30 um.

Due to the low dry matter of the feed extract, the bulk density of the powder was low, i.e. in the range of 0.08 to 0.1 kg/l. A higher dry matter content of e.g. 15 % by weight might have yielded a higher bulk density and larger particles.

It was noted that during the spray drying a major amount of the sotolon present in the aqueous extract was evaporated, which produced a spray dried product with less sotolon off-flavor.

### Example 4

### Viral isolate and growth conditions

All work with viable SARS-CoV-2 were performed in an approved BSL-3 laboratory. Isolates of SARS-CoV-2 ancestral strain (hereafter referred to as wildtype) and variant strains (Table 2) were recovered from patient oropharyngeal swab samples and propagated. 500 µL pharyngeal sample suspended with 4.5 mL 37 °C preheated DMEM with GlutaMAX, 2% HI FBS, 1% PS and 1% Amphotericin B (hereafter infection DMEM (iDMEM)) were added to Vero E6 cells seeded 24 h prior and grown to confluence in T75 culture flasks. The culture flasks were incubated for 1 h on a rocking table followed by adding an additional 15 mL iDMEM. The culture flasks were then incubated for three to five days or until distinct cytopathic effect (CPE) had appeared and all the cells have detached from the flask. The entire content of the culture flasks was aspirated, transferred to a 50 mL centrifuge tube, and centrifuged 5 min. at 450 × g to clear the stock from cell debris. Supernatant was transferred to a new tube and from here aliquoted into 500 µL portions and stored at -80°C. Additionally 200 µL supernatant were diluted 10^3 times in iDMEM and subjected to real time RT-PCR in duplicates.

Table 1 - Virus used in the experiments throughout the thesis. Asterisk (*) implies that the strain was isolated from patient oropharyn-geal swap samples.

**Table 1**

| **Name** | **Strain** | **Reference** |
|---|---|---|
| Wildtype | SARS-CoV-2 (Ancestral strain) | This study* |
| Alpha | SARS-CoV-2 cluster B.1.1.7 variant | This study* |
| Beta | SARS.CoV-2 cluster B.1.351 variant | This study* |
| Delta | SARS-CoV-2 cluster B.1.617.2 variant | This study* |
| Mink | SARS-CoV-2 cluster N439K/Y453F variant | This study* |
| Omicron | SARS-CoV-2 cluster B.1.1.529 variant | This study* |

### Plaque Assay

Quantification of viable SARS-CoV-2 was performed in high-sensitivity plaque assays using Vero E6 cells. Approximately 1×10^6 Vero E6 cells per well were seeded in 6-well microtiter plates and incubated overnight to allow establishment of a confluent monolayer in accordance with section 3.1. After nearly 24 h the monolayer of Vero E6 cells were ready for infection with SARS-CoV-2 containing samples.

Prior to infection, virus containing samples were prepared and diluted, if necessary, in iDMEM, making sure it was possible to count the plaque forming units (PFU) in one of the dilutions. Preparation of the various SARS-CoV-2 samples are explicated in separate sections. If using SARS-CoV-2 stock or virus containing samples stored at -80°C, the samples were thawed in a 37°C water bath prior to use.

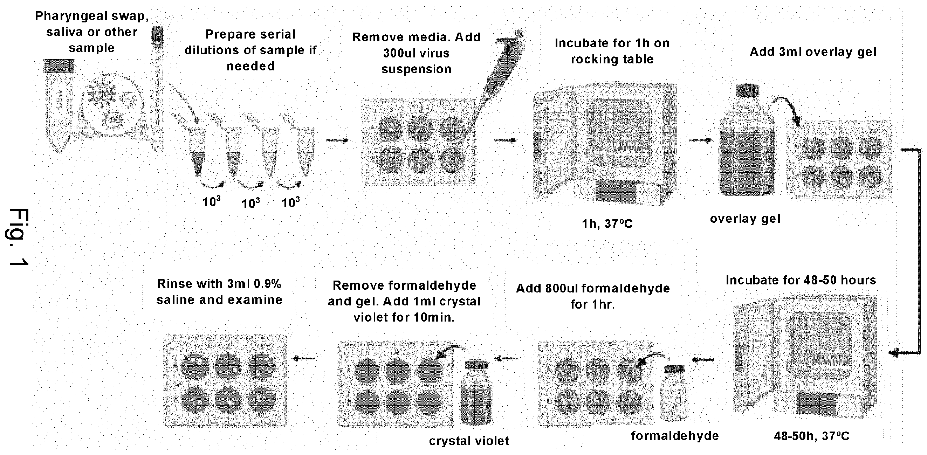

cDMEM was removed from the Vero E6 cells and 300µL virus suspension was placed onto the cell layer. The 6-well microtiter plate was incubated for 1h on a rocking table to prevent the cells from drying out. Meanwhile, overlay medium was prepared. DMEM with GlutaMAX, 3% HI FBS, 1% PS and 1% Amphotericin B (hereafter gel DMEM (gDMEM)) was heated to 48°C in a water bath. 3% agarose in DMEM with GlutaMAX was liquified in the microwave oven for 1-2min. One part liquified 3% agarose was mixed with three parts 48°C gDMEM. Unless otherwise specified, the infection solution remained in the wells when applying gel overlay. 3mL gel overlay was gently placed onto the infected Vero E6 cells and allowed to cool for approximately 5min. before the microtiter plate was incubated for 48-52h, unless otherwise specified. 800pL 10% neutral buffered formalin solution (formaldehyde) (Sigma-Aldrich Cat. No. HT5011) was placed onto the overlay gel to fixate the infected cell layer for at least 1 h and maximum 24h. Subsequently, excess formaldehyde was aspirated and discarded, and the gel overlay was gently removed using a cell lifter. 1mL 10% Gram's crystal violet solution (Sigma-Aldrich Cat. No. 94448) was added to the fixated cells and left for 2min before the cells were rinsed with 3mL 0.9% saline and allowed to dry. The microtiter plate with stained cell layers was examined, photographed and PFU was counted as shown in Fig. 1, which is an illustration of the high-sensitivity plaque assay method. Based on the Cq value of the SARS-CoV-2 containing samples they were diluted to a fitting concentration. cDMEM was removed from the 6-well plates and 300pL sample was added to each well. The plates were incubated for 1h on a rocking table. 3mL overlay gel was applied to each well and the plates were incubated for 48-50h before fixation with 800pL formaldehyde for 1h. Afterwards excess formaldehyde was removed along with the gel overlay. The infected cell layer was stained with 1mL crystal violet for 10min. followed by rinsing with 3mL 0.9% saline solution and examination.

### Example 5

### Inactivation of SARS Covid-19 vira

The fenugreek extract powder used was specifically extracted from fenugreek plant seeds in accordance to a patented method by Jens Steen Olsen in patent DK2155222T3. The extraction was performed elsewhere and provided by QUR Medical ApS. Dissolution of fenugreek always took place approximately 24h before the experiment and stored at 4°C unless otherwise stated.

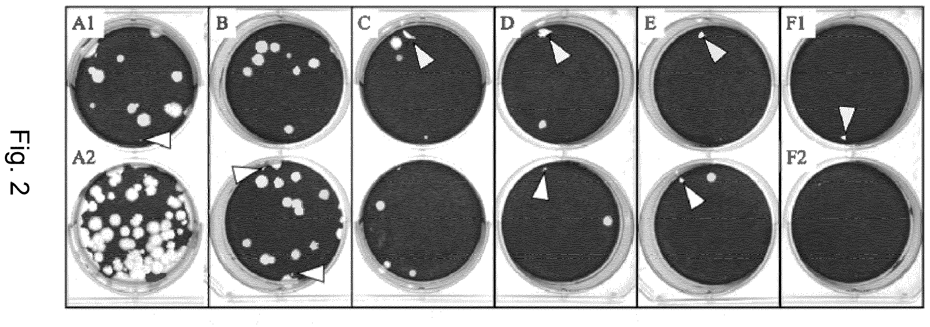

Fenugreek extract powder was dissolved in iDMEM to a concentration of 13.33 mg/mL. SARS-CoV-2 wildtype stock with a concentration of 2×10^6 PFU/mL was mixed 1:9 with dissolved fenugreek extract and incubated. At times 10, 20, 30 and 40min. post incubation 100µL virus suspension was aspirated and diluted in 37°C preheated iDMEM to a concentration of 200 PFU/mL. 300µL diluted sample was added to Vero E6 cell layer in 6-well microtiter plates in duplicates. The microtiter plates were incubated for 1 h before applying a gel overlay, incubated for 48-50h, fixed and stained as described in Example 4. A positive control with both virus and fenugreek, a negative control with fenugreek and without virus, and a negative control without both fenugreek and virus were established at time 0 after incubation alongside the experiment. Fig. 2 illustrates the duplicated plaque assay results, wherein positive control is diluted 10^5 times (A1), positive control diluted 10^4 times (A2), SARS-CoV-2 is treated with fenugreek for 10min. (B), SARS-CoV-2 is treated with fenugreek for 20min. (C), SARS-CoV-2 is treated with fenugreek for 30min. (D), SARS-CoV-2 is treated with fenugreek for 40 min. (E), negative control without fenugreek or virus (F1), and negative control with fenugreek and without virus (F2). White arrowheads show damage in the cell layer caused by the cell lifter upon removing the gel overlay, and are not plaques.

In a second example of Inactivation of SARS Covid-19 vira, the powder according to example 3 was used in an aqueous 0.25% solution in a plaque-forming unit (PFU) study. The study is used to describe the number of virus particles capable of forming plaques per unit volume after treatment with the aqueous solution for a certain time.

The numbers of plaque-forming units are measured in vitro reaction mixtures by standard methods (Methods Enzymol. 1981;78(Pt A):315-25. Authors. R R Rueckert, M A Pallansch). More specifically, the extract powder was dissolved to 2.5% in DMEM + 2% FBS on the same day as the experiment. 100µl virus stock (concentration 2×10⁶ PFU/ml) was added to the powder solution and mixed. Every 10 minutes for 40 minutes 100µl virus suspension was extracted and diluted 10^4 times in DMEM + 2% FBS. 300µl of the diluted virus solution was added to Vero E6 cells and cultured in a plaque assay. In parallel, control plaque assays were conducted in which the powder solution without virus is tested with the Vero E6 cells. The control assay did not show toxic effects on the VERO E3 cells.

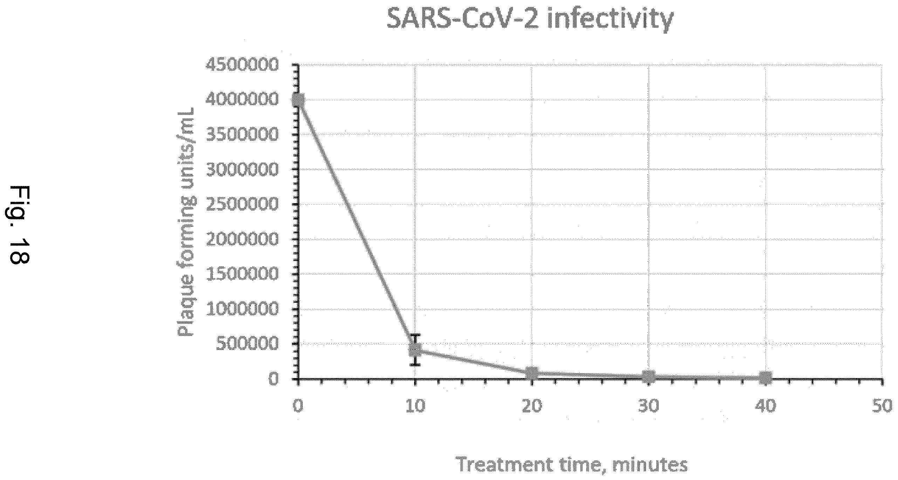

After cultivation the number of surviving virus particles are estimated. The results are shown in Fig. 18.

Fig. 18 shows that the amount of PFU decreases dramatically when treated for 10 minutes with the 0.25% aqueous solution. After treatment for 40 minutes the ability of the SARS-CoV-2 virus to form PFU was reduced by 99.6%.

### Example 6

### Testing of centrifuged fenugreek extract

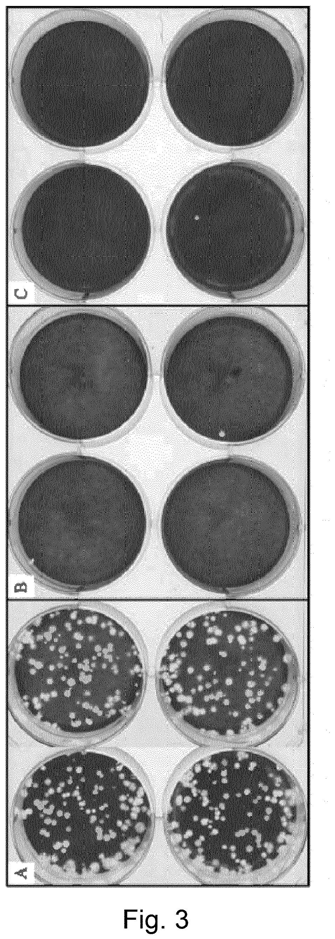

Fenugreek extract powder was dissolved in iDMEM to a concentration of 13.33 mg/mL. From the fenugreek solution a portion was centrifuged at 2.000 xg for 10 min. The supernatant was aspirated to a new tube and the pellet was discarded. SARS-CoV-2 wildtype stock with a concentration of 3.5×10^5 PFU/mL was mixed 1:9 with both fenugreek solution and fenugreek supernatant solution and incubated for 30 min. Post incubation the treatment mixtures were diluted 10^2 times in 37°C preheated iDMEM and 300pL were added to Vero E6 cell layer in 6-well microtiter plates in four replicates. The microtiter plates were incubated for 1 h before applying the gel overlay, incubate, fixate, and stain as described in Example 4. Fig. 3 illustrates the plaque assay results of the four replicates wherein positive control is diluted 10^3 times (A), SARS-CoV-2 is treated with fenugreek supernatant solution post centrifugation (B), and SARS-CoV-2 is treated with fenugreek for (C).

### Example 7

### Testing lozenges containing fenugreek extract

Both a lozenges with fenugreek extract (active lozenge) and a lozenges without fenugreek (placebo lozenge) were made and provided by QUR Medical ApS. The composition of each lozenge is listed in Table 2.

**Table 2**

| | | **Active lozenge** | **Placebo lozenge** |
|---|---|---|---|
| **Ingredient** | **Specification** | **mg/lozenge** | **mg/lozenge** |
| Fenugreek extract powder | Jens Olsen | 24.00 | 0.00 |
| Menthol | T3764 | 1.33 | 1.33 |
| Peppermint | T4603 | 9.50 | 9.50 |
| Syloid AL-1 | E 551 | 4.00 | 4.00 |
| Magnesium stearate, veg. | E 470b | 11.00 | 11.00 |
| Sorbitol granulate | E 420i | 1000.00 | 1000.00 |

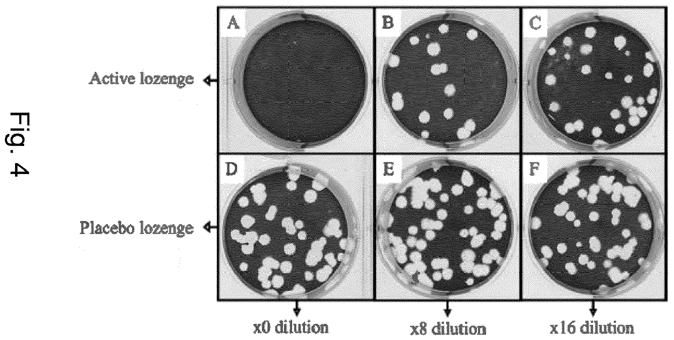

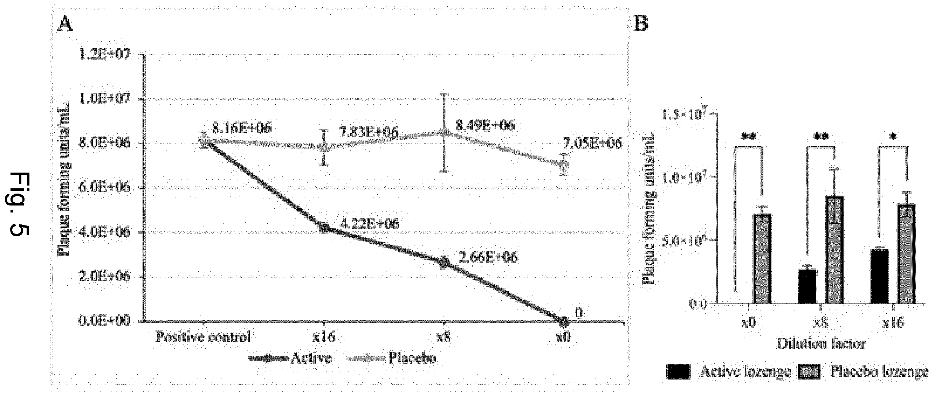

One lozenge was dissolved in 1 mL iDMEM. 200µL iDMEM with dissolved lozenge was mixed with 200µL SARS-CoV-2 wildtype stock with a concentration of 8×10^6 PFU/mL. Hereby the treatment mixture had a fenugreek concentration of 12mg fenugreek powder per mL. Treatment mixture was incubated for 30min. Post incubation the treatment mixture was diluted 5×10^2 times in 37°C preheated iDMEM and 300pL was added to Vero E6 cell layer in 6-well microtiter plates. The microtiter plates were incubated for 1h before applying the gel overlay, incubating, fixing, and staining as described in Example 4. Fig. 4 illustrates one of each triplicate of each fenugreek concentration in the plaque assay, wherein plaque forming units (PFU) of SARS-CoV-2 are treated in vitro with the active and placebo lozenges. A-C illustrates the effect of active lozenge solution with a concentration of 12, 1.5, and 0.75 mg/mL fenugreek, respectively. D-F illustrates the placebo lozenge without fenugreek diluted 0, 8 and 16 times, corresponding to the dilutions in A, B, and C, respectively. PFUs were counted from the plaque assay and illustrated in Fig. 5, wherein (A) is a plot illustrating the SARS-CoV-2 survival at different concentrations of fenugreek treatment. Visualized are the active lozenge com-pared to the placebo lozenge diluted times 0, 8 and 16, respectively. (B) Shows significant decrease in SARS-CoV-2 survival when testing the active lozenge against SARS-CoV-2 (x0: p=0.0023, x8: p=0.0049, and x16 p=0.0277). Results were analysed by two-way ANOVA followed by Sidak's multiple comparison test. Data are presented as mean ± SD.

In a second example of Inactivation of SARS Covid-19 vira using lozanges, astock solution was prepared by dissolving 209 mg of the tablets produced in example 2 in 400 ml water. The stock solution was diluted 2 times, 4 times, 8 times, 16 times and 10⁵ times. The stock and the dilutions were tested on SARS COVID-19 vira and placebo. The results are indicated in the table below.

**Table 3:**

| **Active** | | | **Placebo** | | |
|---|---|---|---|---|---|
| **Dilution** | **PFU/300µL** | **PFU/mL** | **Dilution** | **PFU/300µL** | **PFU/mL** |
| Non-diluted | 2 | 399600 | Non-diluted | 20 | 3996000 |
| x2 | 3 | 599400 | x2 | 27 | 5394600 |
| x4 | 5 | 999000 | x4 | 23 | 4595400 |
| x8 | 12 | 2397600 | x8 | 22 | 4395600 |
| x16 | 10 | 1998000 | x16 | 19 | 3796200 |
| Stock 10^5 | 14 | 4662000 | Stock 10^5 | 14 | 4662000 |

The table shows that a dose-response dependent infectivity is present as the number of PFU decreases when the concentration of the fenugreek extract increases. The placebo or control tests does not show the same dose-response dependency.

### Example 8

### Vero E6 cell viability test

### Human cell line and growth conditions

Vero E6 cells (African green monkey kidney cell clones) (ATCC^{®} CRL-1586^{™}) were kept in T75 cell culture flasks (Thermo Scientific^{™} Cat. No. 156499) with Dulbecco's modified Eagle Medium (DMEM) with GlutaMAX, 10% heat-inactivated fetal bovine serum (HI FBS) and 1% Penicillin-Streptomycin (PS) (10.000 Units/ml Penicillin, 10.000 ug/ml Streptomycin) (GibcoTM Cat. No. 15140122) (hereafter complete DMEM (cDMEM)). The cells were always incubated in a 37°C humidified atmosphere containing 5% CO2, unless stated otherwise. For experiments, Vero E6 cells were seeded 24 h prior to form a monolayer in 6-well plates (Thermo Scientific^{™} Cat. No. 140675) or in T25 culture flasks (Thermo Scientific^{™} Cat. No. 156367). cDMEM was always aspirated prior to use of 6-well plates and T25 culture flasks.

Prior to cell split, Vero E6 cells were washed three times with sterile Phosphate buffered saline (PBS) before treatment with 5mL trypsin for 5min., to break the attachment of Vero E6 cells to the surface of the culture flask. 5mL cDMEM was added to the cell culture flask to stop the effect of trypsin. The cells were transferred to a 15 mL centrifuge tube and centrifuged for 2min. at 200 × g. Supernatant was discarded, and cells were resuspended in 6mL cDMEM. For 6-well plates 1 mL resuspended cells were seeded in each of the 6 wells and incubated overnight (ON) to form a monolayer. For T25 culture flasks 2 mL resuspended cells were seeded in a T25 culture flask with an additional 3mL cDMEM and incubated ON to form a monolayer.

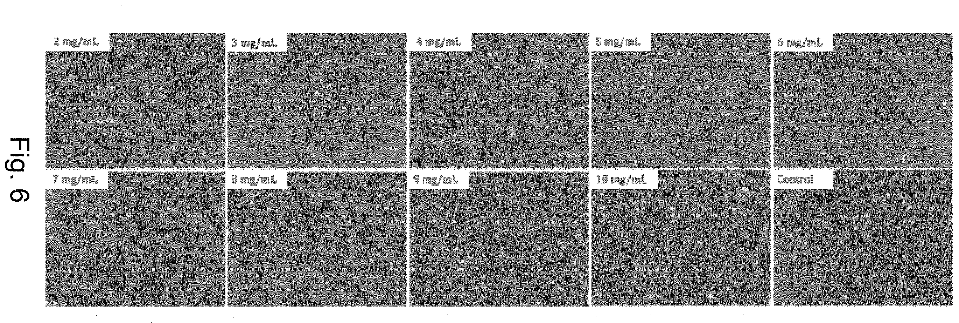

Vero E6 cell layers in a 24-well plate were treated with fenugreek at different concentrations to assess potential cytotoxic effects. Fenugreek extract powder was dissolved in iDMEM to a concentration of 12 mg/mL, from which a dilution row was performed. Dilutions of fenugreek ranging from 1 mg/mL to 12 mg/mL with an interval of 0.5 mg/mL were analyzed in the 24 well plate. Every day for four days the cell layers were inspected manually using a brightfield microscope and pictures were taken on the fourth day after the wells were washed three times with PBS. Fig. 6 shows representative images captured on the fourth day. The images in Fig. 6 shows an excerpt of the brightfield microscope pictures of Vero E6 cell layers treated with different fenugreek concentrations on the fourth day. The minimum concentration displayed is 2 mg/mL, as lower concentrations displayed no effects on the cell layer. The maximum concentration displayed is 10 mg/mL as the cell layers treated with higher concentrations displayed cytopathic effect. The interpretation is thus not solely based on what is shown.

### Example 9

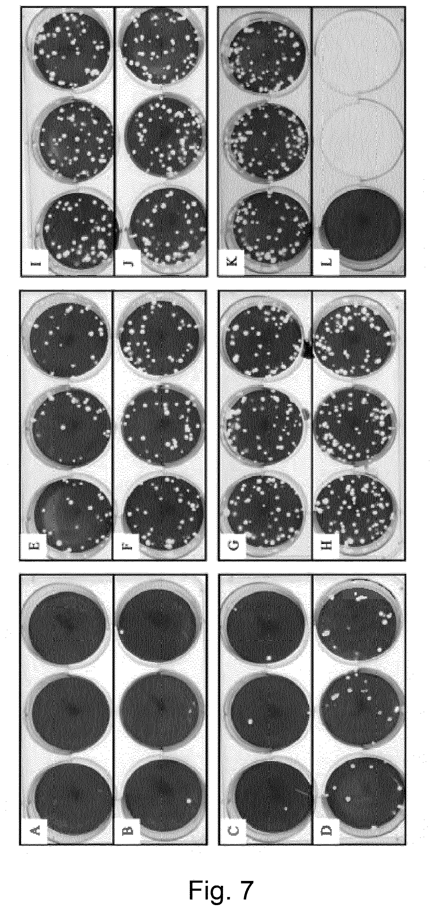

Determining 50% inhibitory concentration for fenugreek SARS-CoV-2 wildtype stock with a concentration of 4×10^5 PFU/mL was mixed 1:9 with fenugreek dissolved in iDMEM yielding final fenugreek concentrations of 12, 6, 3, 1.5, 0.75, 0.375, 0.188, 0.0938, 0.0469 and 0.0234 mg/mL, respectively, and incubated for 30 min. The treated virus samples were diluted 10^2 times in 37°C preheated iDMEM. 300 µL were spotted onto Vero E6 cells in 6-well plates in triplicates and incubated for 1 h on a rocking table, before the gel overlay was applied to each well. Wells were incubated, fixated, and stained as described in Example 4. Triplicates of each concentration were produced in the plaque assay (Fig. 7), wherein Vero E6 cells are infected with fenugreek treated SARS-CoV-2. Different concentrations of fenugreek extract used for treatment was performed in triplicates. The fenugreek concentrations used to treat SARS-CoV-2 for 30 min. were: (A)=12 mg/mL, (B)=6 mg/mL, (C)=3 mg/mL, (D)=1.5 mg/mL, (E)=0.75 mg/mL, (F)=0.375 mg/mL, (G)=0.1875 mg/mL, (H)=0.09375 mg/mL, (I)=0.046875 mg/mL, (J)=0.0234375 mg/mL, (K)=positive control without fenugreek, (L)=negative control without SARS-CoV-2 and fenugreek.

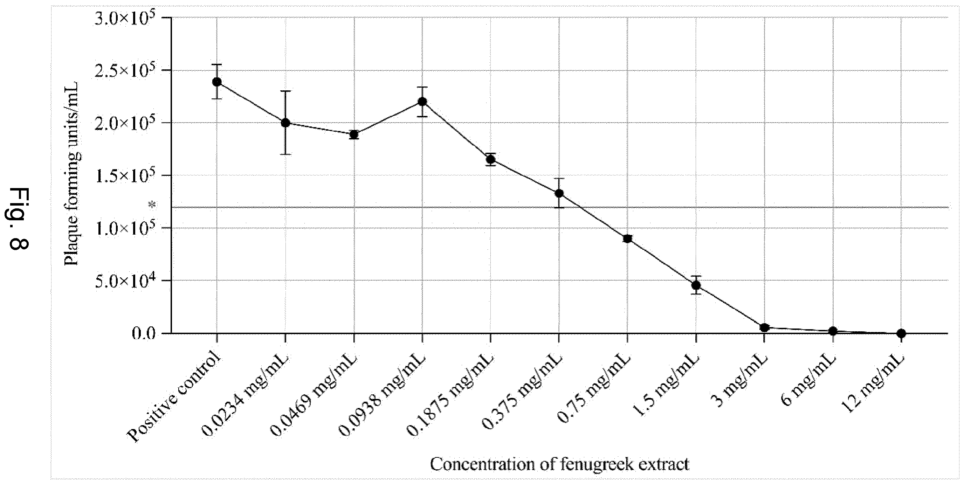

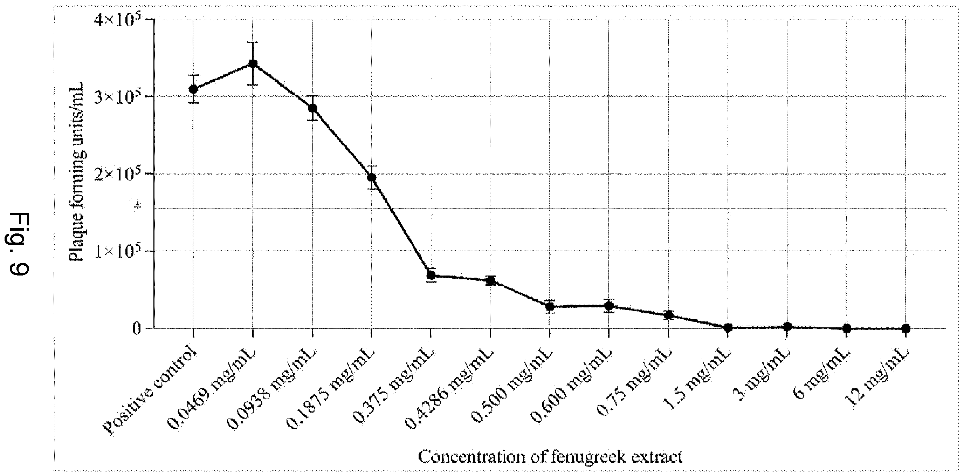

The mean values of the counted PFU from Fig. 7 is shown as graphical presentation in Fig. 8. The red line and asterisk indicate half of the PFU/mL for the control. The intersection with the graph and the red line indicates the theoretical concentration of fenugreek, which would inhibit SARS-CoV-2 by 50%. The experiment was performed twice, with additional dilutions between 0.75 and 0.375 mg/mL the second time Fig. 9.

### Example 10

### Binding mode of fenugreek

Three concurrent experiments were performed in T25 culture flasks with confluent Vero E6 cell layers, one serving as control, one with fenugreek treated cells and one with fenugreek treated virus.

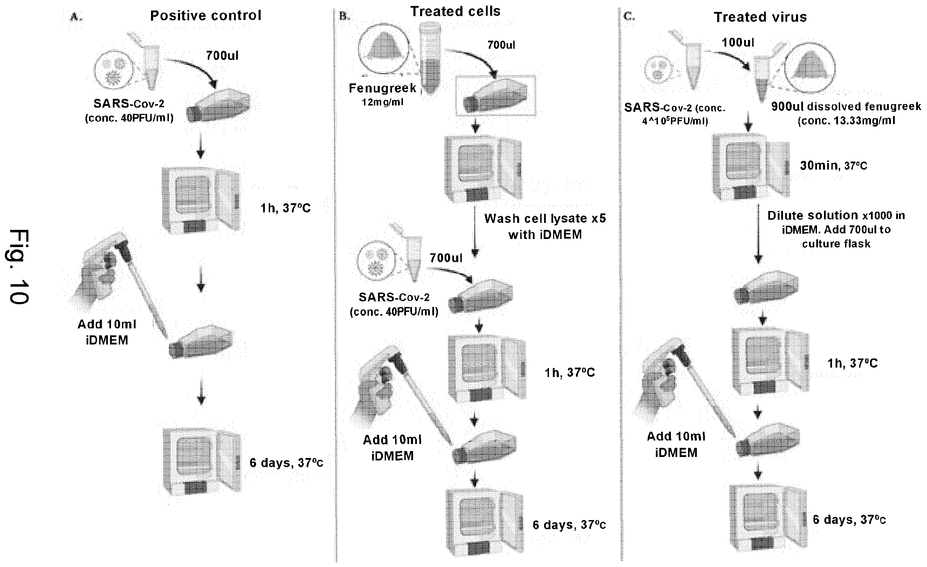

For the positive control setup (Fig. 10A) SARS-CoV-2 wildtype stock with a concentration of 4×10^5 PFU/mL was diluted 10^4 times in 37°C preheated iDMEM yielding a concentration of approximately 40 PFU/mL. 700pL diluted SARS-CoV-2 was added to the Vero E6 cell layer, incubated for 1h on a rocking table, followed by adding an additional 10mL 37°C preheated iDMEM. Culture flasks were incubated for 6 days.

For the culture flasks with a fenugreek treated cell layer (Fig. 10B), powdered fenugreek was dissolved in iDMEM to reach a concentration of 12 mg/mL approximately 24h post experiment. 700uL dissolved fenugreek was added to the Vero E6 cell layer and incubated for 30min. on a rocking table. Post incubation the cell layer was washed five times with 37°C preheated iDMEM followed by adding 700pL wildtype SARS-CoV-2 diluted 10^4 times (as described for the positive control) and incubation for 1h on a rocking table. Hereafter, an additional 10mL 37°C preheated iDMEM were added to the culture flasks and they were incubated for 6 days.

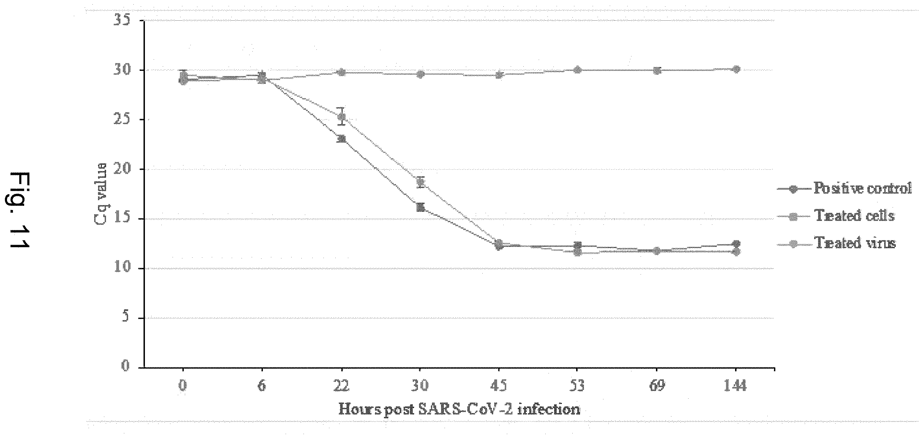

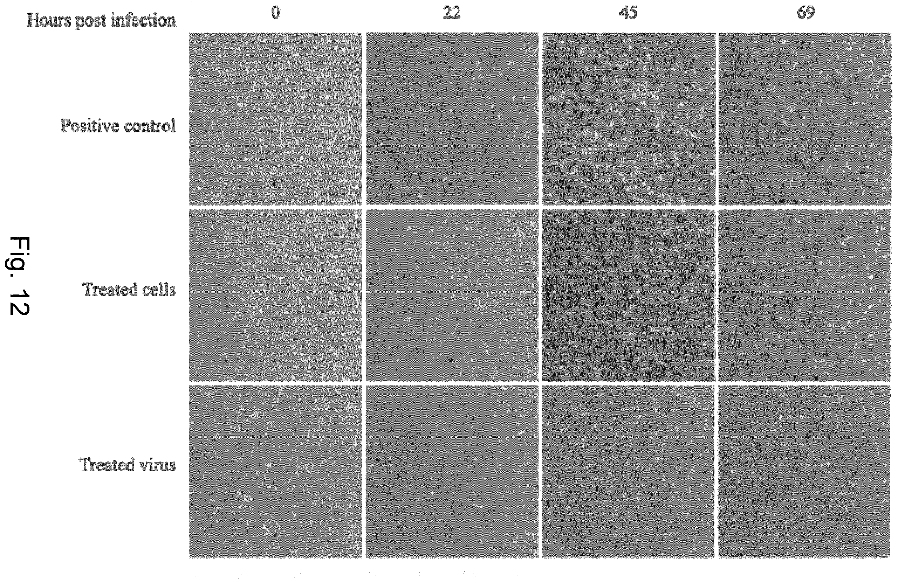

For the culture flask where the cell layer should be infected with fenugreek treated SARS-CoV-2 (Fig. 10C), powdered fenugreek extract was dissolved in iDMEM to reach a concentration of 13.33 mg/mL. SARS-CoV-2 wildtype stock with a concentration of 4×10^5 PFU/mL was mixed 1:9 with dissolved fenugreek, yielding a final fenugreek concentration of 12 mg/mL and incubated for 30min. Post incubation the treatment mixture was diluted an additional 10^3 times in 37°C preheated iDMEM and 700pL was added to the Vero E6 cell layer. Culture flasks were incubated for 1 h on a rocking table, followed by adding an additional 10 mL 37°C preheated iDMEM. Culture flasks were incubated for 6 days. For all culture flasks 650 µL supernatant were aspirated and stored at -20°C before being subjected to real time RT-PCR at 0, 6, 22, 30, 45, 53, 69 and 144h post incubation (Fig. 11). Furthermore, pictures of the cell layers were taken using Moticam X (Motic ^{©}) on a brightfield microscope to observe CPE (Fig. 12).

### Example 10

Electron Microscopy of Fenugreek Treated SARS-CoV-2 Infection Electron microscopy (EM) was performed on two types of SARS-CoV-2 infected Vero E6 cell layers and fixated after both 1 and 16h of infection. For the first setup Vero E6 cells were seeded on membrane inserts in 6-well plate 24h prior to infection. SARS-CoV-2 wildtype stock with a concentration of 5×10^5 PFU/mL was treated in a ratio 1:9 with 13.33 mg/mL fenugreek dissolved in iDMEM for 30min. cDMEM was removed from the wells and 3 mL treated SARS-CoV-2 solution were added to each well, resulting in a final concentration of 5×10^4 PFU/mL, and incubated for 1h on a rocking table. The wells were incubated for an additional 1 and 16h, respectively, before being emptied and fixated with 1.5 mL 2% glutaraldehyde (GA) fixative for 1h at room temperature (RT). The membrane inserts were washed with tris buffered saline (TBS) before being stained with osmium tetroxide for 1h. Afterwards, the stained membrane inserts were washed three times with PBS and cut out of the insert before the membrane was rolled around itself and embedded in a gelatin capsule ready for EM.

For the second setup SARS-CoV-2 wildtype stock with a concentration of 5×10^5 PFU/mL was treated in a ratio 1:9 with 13.33 mg/mL fenugreek dissolved in iDMEM for 30min. Vero E6 cells in T25 culture flasks were infected with 5 mL fenugreek treated SARS-CoV-2 stock and incubated for 1 h on a rocking table. The culture flasks were incubated for an additional 1 and 16h respectively before aspirating the content, washing the infected cell layers with PBS trice, and adding 1mL 2% GA fixative. The culture flasks were incubated for 1h at RT. Subsequently the cell layer was scraped off using a cell lifter and the entire content of the culture flasks were aspirated into a centrifuge tube, centrifuged at 200 × g for 2min. and the supernatant removed. 5mL of 15% bovine serum albumin dissolved in TBS were added to the cell pellet, mixed gently, and incubated for 30 min. at RT. Afterwards, the resuspended cells are centrifuged at 1.000 × g for 5min. The supernatant was aspirated, and the cell pellet was carefully overlayed with 2% GA fixative in PBS and incubated overnight at 4°C. The solidified pellets were recovered and processed for embedding in epon before EM.

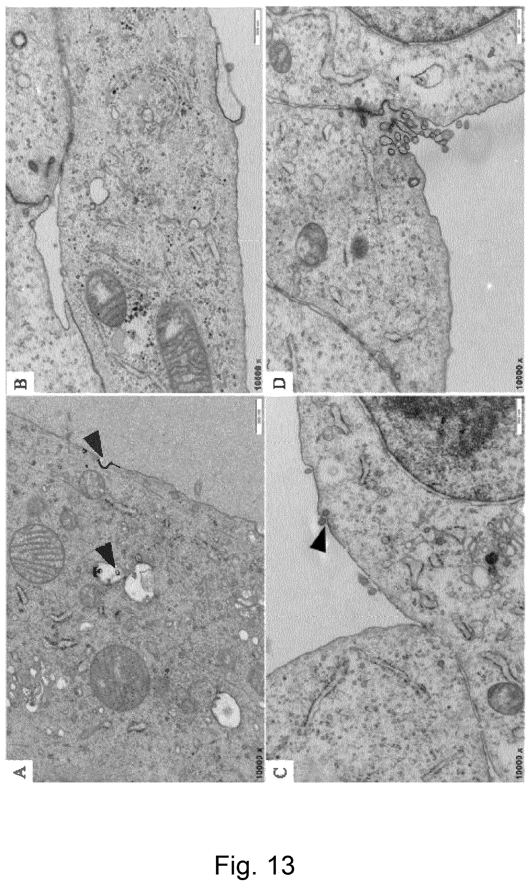

For the infections fixated 1h p.i., it was only possible to identify a few SARS-CoV-2 virus particles using EM. Shown in Fig. 13A-B are cell layers grown in culture flasks and infected with SARS-CoV-2 and fenugreek treated SARS-CoV-2, respectively. Fig. 13C-D shows the cell layers grown in membrane inserts and infected with SARS-CoV-2 and fenugreek treated SARS-CoV-2, respectively. Black arrowheads indicate SARS-CoV-2 particles infecting the Vero E6 cells.

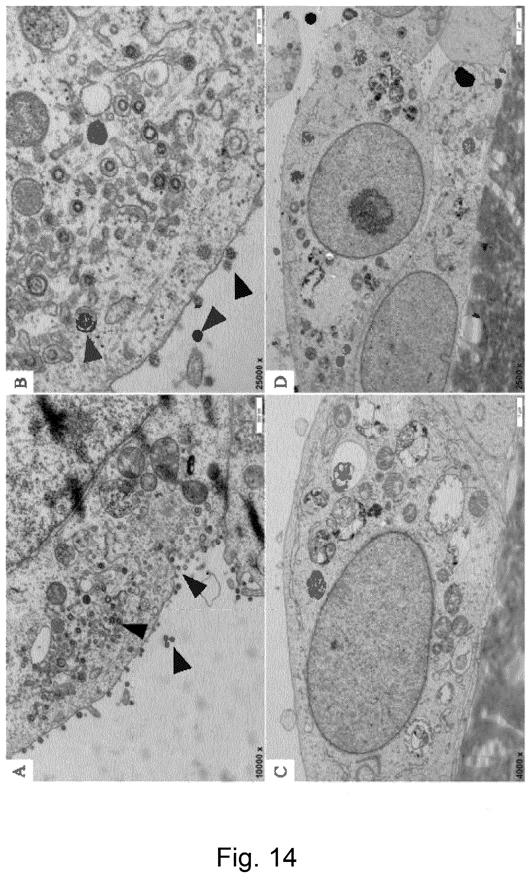

The infections performed on membrane inserts and fixated 16h p.i. are shown in Fig. 14. On the images from the positive control (Fig. 14A-B) black arrowheads indicate some of the numerus SARS-CoV-2 particles either intracellular, extracellular, or bound to the cell membrane. The Vero E6 cells on membrane inserts treated with fenugreek (Fig. 14C-D) showed no sign of SARS-CoV-2 particles.

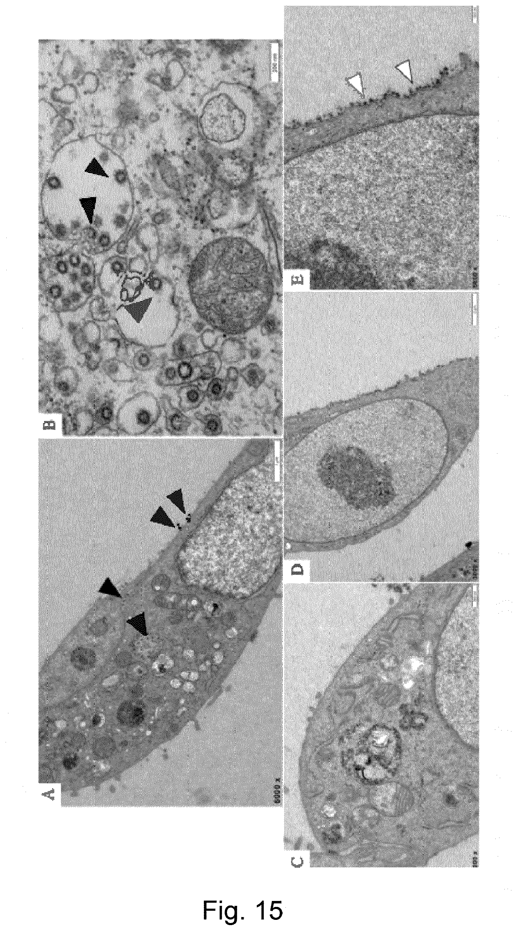

Visualized in Fig. 15 are the infections performed in culture flasks and fixated 16h p.i. On the images from the positive control (Fig. 15A-B) black arrowheads show some of the numerus SARS-CoV-2 either intracellular or bound to the cell membrane. The Vero E6 cells treated with fenugreek (Fig. 15C-E) showed no sign of SARS-CoV-2. However, the white arrowheads in Fig. 15E, which is a closeup picture of Fig. 15D, show possible SARS-CoV-2 particles. How-ever, the closeup picture does not provide further confirmation of whether it is virus particles.

### Example 11

### Applying fenugreek to infected cell layer

Fenugreek extract was dissolved in iDMEM to a concentration of 60 mg/mL. Vero E6 cells grown to a confluent cell layer in 12 T25 culture flasks were infected with 700 µL SARS-CoV-2 wildtype stock diluted 10^4 times and incubated for 1h on a rocking table. Afterwards, three flasks (referred to as control) were plenished with 5mL iDMEM, three flasks (referred to as T0) were plenished with 4mL iDMEM and 1 mL 60 mg/mL fenugreek solution yielding a fenugreek concentration of 12 mg/mL. The remaining six culture flasks (three referred to as T20 and three referred to as T26, respectively) were plenished with 4mL iDMEM. 650 µL supernatant was aspirated from all culture flasks and stored at -20°C, indicating the starting point of infection for all culture flasks. An additional 650 µL iDMEM was added to the T20 and T26 culture flasks and all culture flasks were then incubated for 20h. 20h post infection (p.i.) 1mL 60 mg/mL fenugreek solution was added to the T20 culture flasks, yielding a fenugreek concentration of 12 mg/mL. 650 µL supernatant was aspirated from all culture flasks and stored at -20°C. An additional 650pL iDMEM was added to the T26 culture flasks and all culture flasks were incubated. 26 h p.i. 1mL 60 mg/mL fenugreek solution was added to the T26 culture flasks, yielding a fenugreek concentration of 12 mg/mL. 650pL supernatant was aspirated from each culture flask and stored at -20°C. The culture flasks were then incubated and at 44 h and 68 h p.i., respectively, 650pL supernatant was aspirated from each culture flask and stored at -20°C. Afterwards, all samples were thawed at RT. Samples containing fenugreek were centrifuged at 450 × g for 5 min. and the supernatant was diluted 2 times in iDMEM. All samples were subjected to RT-PCR and Cq values were evaluated.

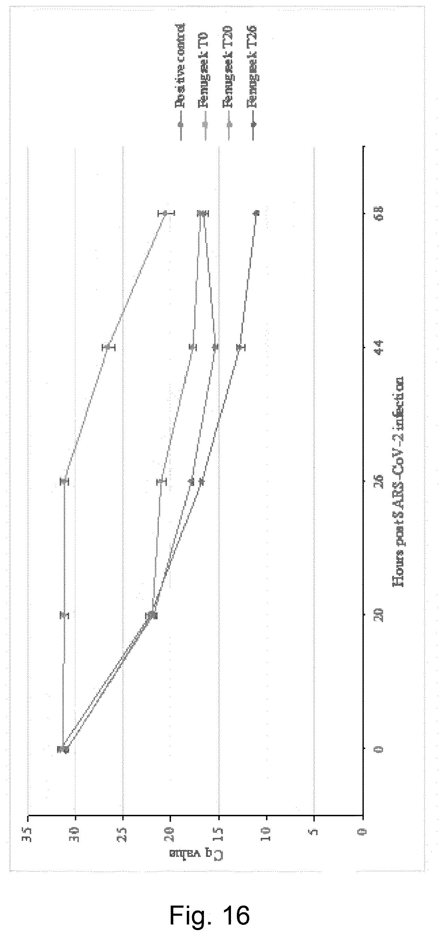

Illustrated in Fig. 16 are culture flasks serving as positive controls, culture flasks to which fenugreek extract was added immediately after infection (T0), culture flasks to which fenugreek extract was added 20h p.i. (T20), and culture flasks to which fenugreek extract was added 26 h p.i., (T26).

### Example 11

### Fenugreek against sars-cov-2 variants

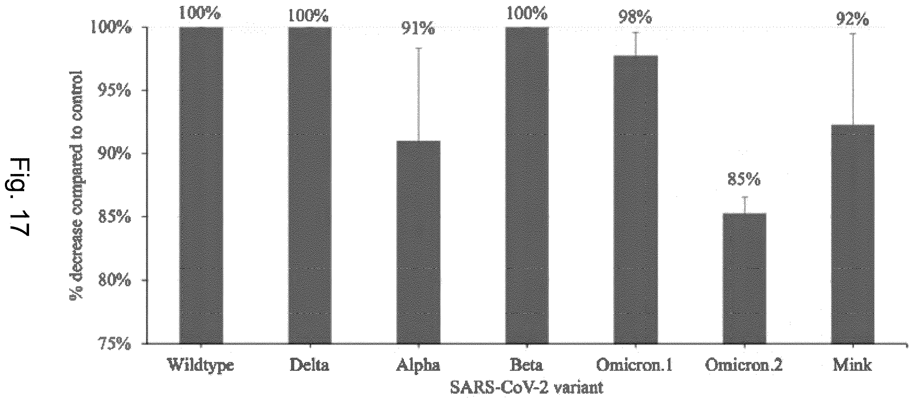

Fenugreek extract was dissolved in iDMEM to a concentration of 13.33 mg/mL. SARS-CoV-2 and SARS-CoV-2 variants (see Table 4 for stock concentrations) were mixed 1:9 with dissolved fenu-greek and incubated for 30 min. Afterwards, the samples were diluted in 37°C preheated iDMEM to reach the final dilution factor listed in Table 4. The SARS-CoV-2 treated samples were tested in the plaque assay as described in section 3.3 with exception for the incubation period before fixation. For each SARS-CoV-2 strain the incubation period before fixation is listed in Table 4. In Fig. 17 the decrease in SARS-CoV-2 or SARS-CoV-2 variants is illustrated in percentage.

**Table 4**

| **Name** | **SARS-CoV-2 strain** | **Stock concentration** | **Dilution factor** | **Incubation** |
|---|---|---|---|---|
| Wildtype | SARS-CoV-2 | 4 × 10⁵ PFU/mL | 1.000x | 2 days |
| Alpha | Cluster B.1.1.7 variant | 8.21 × 10⁴ PFU/mL | 750x | 3 days |
| Beta | Cluster B.1.351 variant | 5.58 × 10⁴ PFU/mL | 1.000x | 3 days |
| Delta | Cluster B.1.617.2 variant | 7.1 × 10⁴ PFU/mL | 2.500x | 3 days |
| Omicron | Cluster B.1.1.529 variant | 1 × 10⁶ PFU/mL | 5.000x | 3 days |
| Mink | Cluster N439K/Y453F variant | 1.33 × 10⁵ PFU/mL | 2.500x | 3 days |

### Example 12

### Statistical analysis for Examples 1 - 11

Statistical analysis was performed using GraphPad Prism version 9.1.2 (GraphPad Software, USA). Methods to test for normal distribution were Anderson-Darling test, D'Agostino & Pearson test, Shapiro-Wilk test, and Kolmogorov-Smirnov test. If passing the normality test one-way or two-way ANOVA and Šídák's multiple comparison test was performed. All data were presented as mean ± standard deviation (SD). P values <0.05 are considered statistically significant and are denoted as p<0.05 = *, p<0.01 = **, p<0.001 = ***, p<0.0001 = ****.

### Example 13

### EXPLORATORY CLINICAL STUDY

### EXPLORATORY STRATEGY

The study was based on testing lozenges with fenugreek produced and provided by the company QUR Medical ApS. The study aim was to investigate the effect of fenugreek lozenges in volunteers immediately or no later than three days after testing positive for COVID-19. The following lozenges were tested in the study: Active lozenge containing 24 mg fenugreek extract, active lozenge containing 240 mg fenugreek extract, and a placebo lozenge.

An oropharyngeal swab sample, taken by trained personnel using the Universal Transport MediumTM (UTM) swabs, and a saliva sample (min. 1 mL) were collected both before and after consumption of the lozenges. The lozenges were to be taken individually and over a time span of 20 min. after the initial oropharyngeal swab and saliva sample were collected. All samples were stored in a cooler once collected and handled in laminar air flow benches once in the BSL-3 laboratory.

### PREPARATION OF OROPHARYNGEAL SWAB AND SALIVA SAMPLES

From the oropharyngeal swab samples 650 µL sample was subjected to RT-PCR serving as 'original Cq value'. Saliva samples were diluted 4 times in UTM to make the samples less viscous for RT-PCR and for the plaque assay. 650 µL diluted saliva sample was subjected to RT-PCR serving as the original Cq value. The remaining sample material from oropharyngeal and saliva samples were stored at -80°C until further analysis.

### ANALYSIS OF OROPHARYNGEAL SWAB AND SALIVA SAMPLES

Based on the original Cq value the sample was diluted according to Table 1 in 37°C preheated Dulbecco's modified Eagle Medium (DMEM) with GlutaMAX, 2% heat-inactivated fetal bovine serum (HI FBS) and 1% Penicillin-Streptomycin (PS) (10.000 Units/ml Penicillin, 10.000 ug/ml Streptomycin) (GibcoTM Cat. No. 15140122) (hereafter complete iDMEM). The samples and its associated dilutions were then ready for plaque assay.

**Table 1 - Dilution of SARS-CoV-2 samples based on Cq values.**

| | | | | |
|---|---|---|---|---|
| Original Cq value | 100 | >29 | 25-28.99 | <24.99 |
| Dilution | No dilution | 100× | 1.000× | 10.000× |

### RESULTS

A total of 57 volunteers participated in the exploratory clinical study. The outcome of the study involves the same types of data from two test groups: 19 participants receiving 24mg fenugreek lozenges (test group 1) and 38 participants receiving 240mg fenugreek lozenges (test group 2). Four participants from test group 1 received placebo lozenges in addition to active lozenges. The results from the placebo lozenges showed no effect and the specific results from placebo lozenges is therefore excluded from the following data processing and results.

Among the participants in test group 1, 68.42% were vaccinated with at least 2 doses. The participants filled out a questionnaire regarding the symptoms they had experienced during the SARS-CoV-2 infection. No apparent relation between Cq value and number or types of symptoms could be observed. No follow-up on the participants symptoms post treatment were conducted.

To account for excessive variation in Cq values between pre- and post-samples, and to account for uncertain plaque forming units (PFU) counted in the plaque assays, the following exclusion criteria were applied:
- Cq value must not differ more than 5 Cq values between pre- and post-treatment samples
- A minimum of 33.33 PFU/mL should be counted in the plaque assay.

In the following, results are depicted both with and without exclusion criteria.

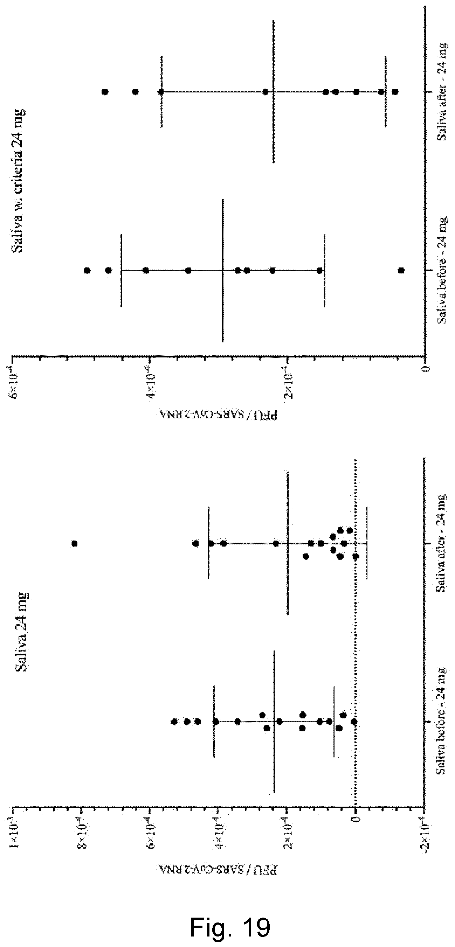

Fig. 19 shows results from saliva samples pre- and post-treatment with 24mg fenugreek lozenges. Four participants were excluded due to negative SARS-CoV-2 RT-PCR saliva samples prior to treatment, and thus the saliva sample size was 15 participants. The mean values of PFU/SARS-CoV-2 RNA in samples post-treatment were 170 lower than in pre-treatment samples. Applying the exclusion criteria, a reduction of 25% in the mean values of PFU/SARS-CoV-2 RNA in samples post-treatment was observed compared to pre-treatment.

Out of the 38 participants in test group 2 only 27 have been analyzed. Among all the participants in test group 2, 81.580 were vaccinated with at least 2 doses. No apparent relation between Cq value and number or types of symptoms was observed.

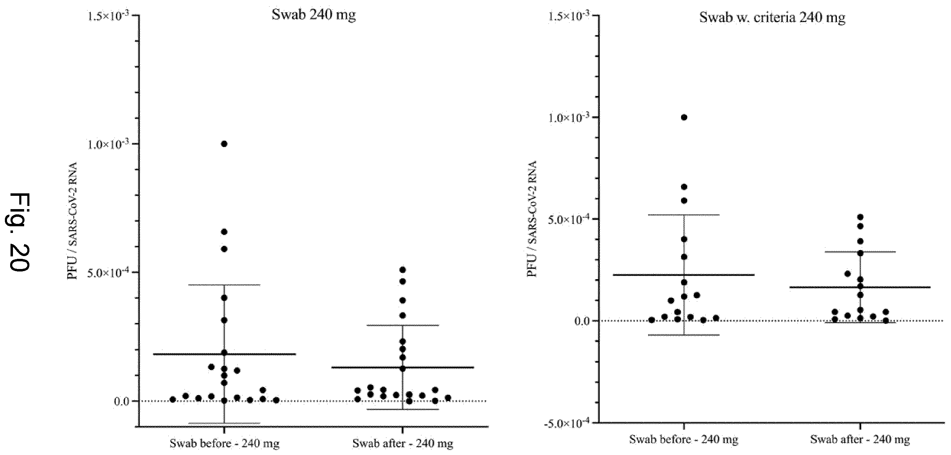

Fig. 20 shows results from oropharyngeal swab samples pre- and post-treatment with 240 mg fenugreek lozenges. Six participants were excluded due to negative SARS-CoV-2 RT-PCR swab samples prior to treatment, and thus the sample size was 21 participants. A reduction of 28% in mean values of PFU/SARS-CoV-2 RNA was observed in samples post-treatment compared to samples taken pre-treatment. If applying the exclusion criteria, there was a reduction of 27% in the mean values of PFU/SARS-CoV-2 RNA in samples post-treatment compared to samples taken pre-treatment.

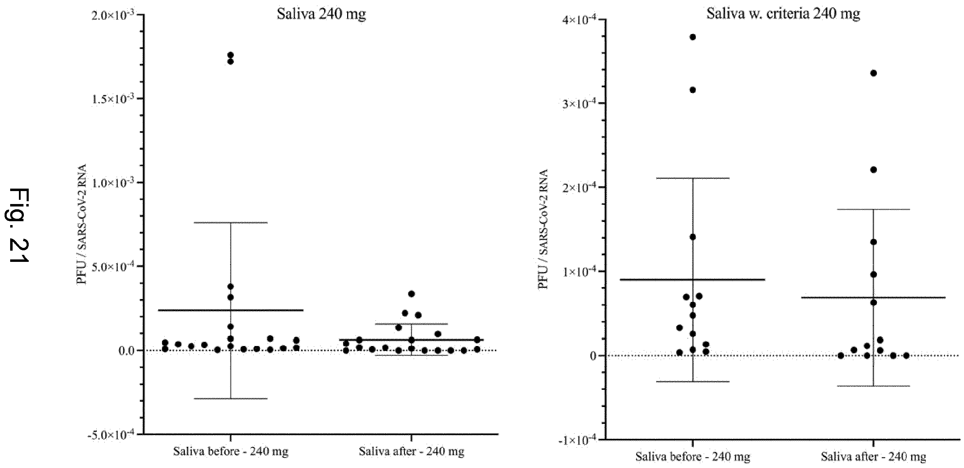

Fig. 21 illustrates saliva sample results pre- and post-treatment. Five participants were excluded due to negative SARS-CoV-2 RT-PCR saliva samples prior to treatment, and thus the sample size was 20 participants. There was a 73% reduction in mean values of PFU/SARS-CoV-2 RNA in samples post-treatment compared to pre-treatment. Applying the criteria, the reduction post treatment in PFU/SARS-CoV-2 RNA was 240.

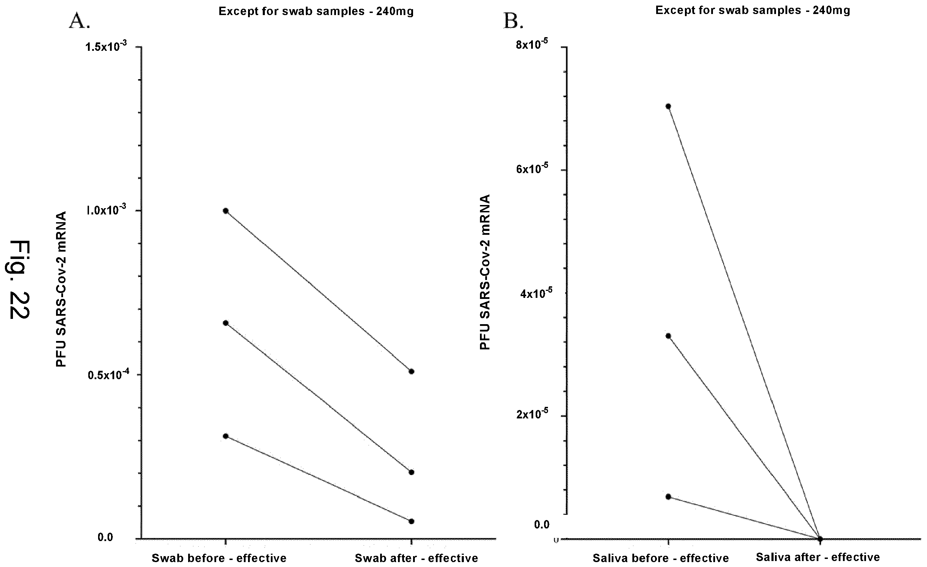

Examples of results for individual persons testing the 240 mg fenugreek lozenge are shown in Fig. 22 A-B.

### Example 14

Anti-HSV and anti-HSV-I effect of a plant extracts.

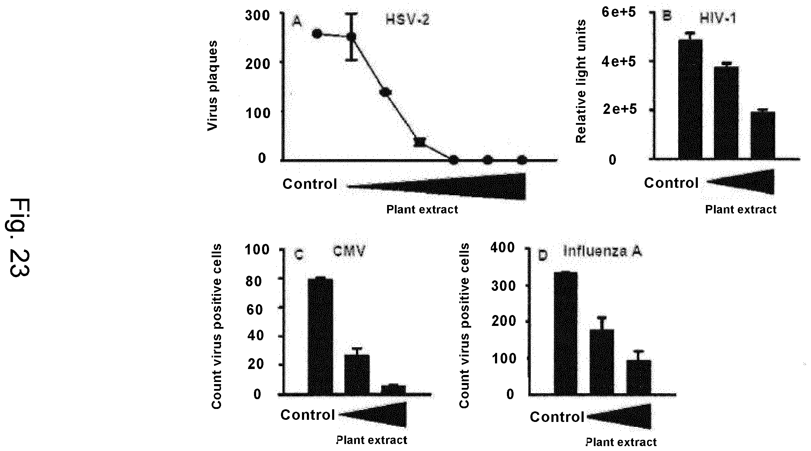

In Fig. 23 diluted plants seed extracts were incubated for 20 min with (A) HSV-2, (B) HIV-1, Influenza A (C), CMV (D), Adenovirus (E) or RSV (F) before adding virus to target cells. Virus quantification was made on day 1-3 by viral plaque assay, reporter assay or immunostain. Representative data of four (A), three (B and C) and two independent experiments (D-F), mean + /- SD of duplicates.

### Example 15

### STATISTICAL ANALYSIS for examples 13 - 14

Statistical analysis was performed using GraphPad Prism version 9.1.2 (GraphPad Software, USA). One-way ANOVA and Šídák's multiple comparison tests were performed. All data were presented as mean ± standard deviation (SD). P values <0.05 are considered statistically significant and are denoted as p<0.05 = *, p<0.01 = **, p<0.001 = ***, p<0.0001 = ****.

## Claims

1. A pharmaceutical composition for use in a method for the treatment of virus-induced disease, wherein the composition comprises an extract of Trigonella foenum-graecum and optionally pharmaceutically acceptable additives wherein the method comprises administration of between 20 mg and 250 mg Trigonella foenum-graecum extract at least once daily.

2. The pharmaceutical composition according to claim 1, wherein the virus is an enveloped virus, Coronavirus, a Simplexvirus, a Cytomegalovirus, an Orthomixovirus, and/or a Retrovirus.

3. The pharmaceutical composition according to claim 2, wherein the virus is a Severe Acute Respiratory Syndrome-related Coronavirus (SARSr-CoV), such as SARS-CoV-1 or SARS-CoV-2, a Herpes Simplex Virus 1 or 2, a Human Betaherpesvirus 5 (HCMV, human cytomegalovirus, HHV-5), an Influenza A, B, C or D virus and/or a Human Immunodeficiency Virus 1 or 2 (HIV-1, HIV-2).

4. The pharmaceutical composition according to claim 3, wherein said SARSr-CoV virus is a SARS-CoV-2 wildtype or an Alpha (B.1.1.7), Beta (B.1.351), Gamma (P.1), Delta (B.1.617.2), Mink (Cluster N439K/Y453F variant), and/or Omicron (B.1.1.529), Epsilon (B.1.427/B.1.429), Zeta (P.2), Eta (B.1.525), Theta (P.3), Iota (B.1.526), Kappa (B.1.617.1), Lambda (C.37), or Mu (B.1.621) genetic lineage variant.

5. The pharmaceutical composition according to any one preceding claim, wherein the disease is COVID-19, cold sores, genital herpes, mononucleosis, pneumonia, influenza (flu) and/or acquired immunodeficiency syndrome (AIDS).

6. The pharmaceutical composition according to any one preceding claim, wherein said pharmaceutical composition is formulated as a solution for spraying, as a topical medication, as a throat lozenge, or as a tablet.

7. The pharmaceutical composition according to any one preceding claim, further comprising bentonite.

8. The pharmaceutical composition according to claim 7, wherein the bentonite comprises 50% by weight or more of smectite.

9. The pharmaceutical composition according to any one of claims 7 or 8, comprising 1:10 to 10:1 by weight dry matter of Trigonella foenum-graecum extract to bentonite.

10. The pharmaceutical composition according to any one preceding claim, wherein a preventive treatment is initiated for a person prior to the contact with another person with symptoms typical of a disease according to claim 6 or with another person who has tested positive for a virus according to any one of claims 1 to 5.

11. The pharmaceutical composition according to any one preceding claim, wherein a component of the Trigonella foenum-graecum extract diminishes the ability of the virus to replicate by diminishing its ability to attach to a host cell, penetrate a host cell, replicate/transcribe its genetic material and/or release from a host cell.

12. The pharmaceutical composition according to any one preceding claim, wherein the component of the Trigonella foenum-graecum extract contacts a component of the virus lipidic membrane.

13. The pharmaceutical composition according to claim 12, wherein the Trigonella foenum-graecum extract component effects a change in the viral lipidic membrane, such as a decrease of structural integrity of said membrane.

14. The pharmaceutical composition according to claim 11, wherein the Trigonella foenum-graecum extract component effects a change on a cellular membrane of a host cell.

## Patentansprüche

1. Pharmazeutische Zusammensetzung zur Verwendung in einem Verfahren zur Behandlung von Virus-induzierter Krankheit, wobei die Zusammensetzung einen Extrakt aus Trigonella foenum-graecum und optional pharmazeutisch verträgliche Zusatzstoffe umfasst, wobei das Verfahren die Verabreichung von zwischen 20 mg und 250 mg Trigonella foenum-graecum-Extrakt mindestens einmal täglich umfasst.

2. Pharmazeutische Zusammensetzung nach Anspruch 1, wobei das Virus ein umhülltes Virus, Coronavirus, ein Simplexvirus, ein Cytomegalovirus, ein Orthomixovirus und/oder ein Retrovirus ist.

3. Pharmazeutische Zusammensetzung nach Anspruch 2, wobei das Virus ein mit dem Schweren Akuten Respiratorischen Syndrom zusammenhängendes Coronavirus (SARSr-CoV), wie SARS-CoV-1 oder SARS-CoV-2, ein Herpes Simplex Virus 1 oder 2, ein Humanes Betaherpesvirus 5 (HCMV, Humanes Cytomegalovirus, HHV-5), ein Influenza A-, B-, C- oder D-Virus und/oder ein Humanes Immunschwächevirus 1 oder 2 (HIV-1, HIV-2) ist.

4. Pharmazeutische Zusammensetzung nach Anspruch 3, wobei das SARSr-CoV-Virus ein SARS-CoV-2-Wildtyp oder ein Alpha (B.1.1.7), Beta (B.1.351), Gamma (P.1), Delta (B.1 .617.2), Mink (Cluster N439K/Y453F-Variante), und/oder Omicron (B.1.1.529), Epsilon (B.1.427/B.1.429), Zeta (P.2), Eta (B.1 .525), Theta (P.3), Iota (B.1.526), Kappa (B.1.6l7.1), Lambda (C.37), oder Mu (B.1.621) genetische Linienvariante ist.

5. Pharmazeutische Zusammensetzung nach einem der vorhergehenden Ansprüche, wobei die Krankheit COVID-19, Fieberbläschen, Genitalherpes, Mononukleose, Lungenentzündung, Influenza (Grippe) und/oder erworbenes Immunschwächesyndrom (AIDS) ist.

6. Pharmazeutische Zusammensetzung nach einem der vorhergehenden Ansprüche, wobei die pharmazeutische Zusammensetzung als Lösung zum Sprühen, als topisches Medikament, als Halsbonbon oder als Tablette formuliert ist.

7. Pharmazeutische Zusammensetzung nach einem der vorherigen Ansprüche, ferner umfassend Bentonit.

8. Pharmazeutische Zusammensetzung nach Anspruch 7, wobei der Bentonit 50 Gew.- % oder mehr Smektit umfasst.

9. Pharmazeutische Zusammensetzung nach einem der Ansprüche 7 oder 8, umfassend 1:10 bis 10:1, bezogen auf die Gewichtstrockenmasse, von Trigonella foenum-graecum-Extrakt zu Bentonit.

10. Pharmazeutische Zusammensetzung nach einem der vorhergehenden Ansprüche, wobei eine präventive Behandlung für eine Person vor dem Kontakt mit einer anderen Person mit typischen Symptomen einer Krankheit nach Anspruch 6 oder mit einer anderen Person, die positiv auf ein Virus nach einem der Ansprüche 1 bis 5 getestet wurde, eingeleitet wird.

11. Pharmazeutische Zusammensetzung nach einem der vorhergehenden Ansprüche, wobei eine Trigonella foenum-graecum-Extraktkomponente die Fähigkeit des Virus zur Replikation verringert, indem es seine Fähigkeit verringert, sich an eine Wirtszelle anzuheften, in eine Wirtszelle einzudringen, sein genetisches Material zu replizieren/transkribieren und/oder sich von einer Wirtszelle zu lösen.

12. Pharmazeutische Zusammensetzung nach einem der vorhergehenden Ansprüche, wobei die Trigonella foenum-graecum-Extraktkomponente eine Komponente der Viruslipidmembran kontaktiert.

13. Pharmazeutische Zusammensetzung nach Anspruch 12, wobei die Trigonella foenum-graecum-Extraktkomponente eine Veränderung der viralen Lipidmembran bewirkt, wie z.B. eine Abnahme der strukturellen Integrität der Membran.

14. Pharmazeutische Zusammensetzung nach Anspruch 11, wobei die Trigonella foenum-graecum-Extraktkomponente eine Veränderung an einer Zellmembran einer Wirtszelle bewirkt.

## Revendications

1. Composition pharmaceutique destinée à être utilisée dans un procédé pour le traitement d'une maladie induite par un virus, dans laquelle la composition comprend un extrait de Trigonella foenum graecum et éventuellement des additifs pharmaceutiquement acceptables, dans laquelle le procédé comprend l'administration d'entre 20 mg et 250 mg d'extrait de Trigonella foenum graecum au moins une fois par jour.

2. Composition pharmaceutique selon la revendication 1, dans laquelle le virus est un virus enveloppé, un coronavirus, un simplexvirus, un cytomégalovirus, un orthomixovirus et/ou un rétrovirus.

3. Composition pharmaceutique selon la revendication 2, dans laquelle le virus est un coronavirus lié au syndrome respiratoire aigu sévère (SARSr-CoV), tel que le SARS-CoV-1 ou le SARS-CoV-2, un virus de l'herpès simplex 1 ou 2, un bêta-herpèsvirus humain 5 (HCMV, cytomégalovirus humain, HHV-5), un virus de la grippe A, B, C ou D et/ou un virus d'immunodéficience humaine 1 ou 2 (VIH-1, VIH-2).

4. Composition pharmaceutique selon la revendication 3, dans laquelle ledit virus SARSr-CoV est un type sauvage de SARS-CoV-2 ou une variante de lignée génétique Alpha (B.1.1.7), Beta (B.1.351), Gamma (P.1), Delta (B.1.617.2), Vison (Cluster N439K/Y453F) et/ou Omicron (B.1.1.529), Epsilon (B.1.427/B.1.429), Zeta (P.2), Êta (B.1.525), Thêta (P.3), Iota (B.1.526), Kappa (B.1.6l7.1), Lambda (C.37) ou Mu (B.1.621).

5. Composition pharmaceutique selon l'une quelconque des revendications précédentes, dans laquelle la maladie est la COVID-19, l'herpès labial, l'herpès génital, la mononucléose, la pneumonie, l'influenza (grippe) et/ou le syndrome d'immunodéficience acquise (SIDA).

6. Composition pharmaceutique selon l'une quelconque des revendications précédentes, dans laquelle ladite composition pharmaceutique est formulée en tant que solution à pulvériser, médicament topique, pastille pour la gorge ou comprimé.

7. La composition pharmaceutique selon l'une quelconque des revendications précédentes, comprenant en outre de la bentonite.

8. Composition pharmaceutique selon la revendication 7, dans laquelle la bentonite comprend 50 % en poids ou plus de smectite.

9. Composition pharmaceutique selon l'une quelconque des revendications 7 ou 8, comprenant 1:10 à 10:1 en poids de matière sèche d'extrait de Trigonella foenum graecum sur bentonite.

10. Composition pharmaceutique selon l'une quelconque des revendications précédentes, dans laquelle un traitement préventif est initié pour une personne avant le contact avec une autre personne présentant des symptômes typiques d'une maladie selon la revendication 6 ou avec une autre personne qui a été testée positive à un virus selon l'une quelconque des revendications 1 à 5.

11. Composition pharmaceutique selon l'une quelconque des revendications précédentes, dans laquelle un composant de l'extrait de Trigonella foenum graecum diminue la capacité du virus à se répliquer en diminuant sa capacité à se fixer à une cellule hôte, à pénétrer dans une cellule hôte, à répliquer/transcrire son matériel génétique et/ou à se libérer d'une cellule hôte.

12. Composition pharmaceutique selon l'une quelconque des revendications précédentes, dans laquelle le composant de l'extrait de Trigonella foenum graecum entre en contact avec un composant de la membrane lipidique virale.

13. Composition pharmaceutique selon la revendication 12, dans laquelle le composant d'extrait de Trigonella foenum graecum effectue un changement dans la membrane lipidique virale, tel qu'une diminution de l'intégrité structurelle de ladite membrane.

14. Composition pharmaceutique selon la revendication 11, dans laquelle le composant d'extrait de Trigonella foenum graecum effectue un changement sur une membrane cellulaire d'une cellule hôte.
